# EUROPEAN PATENT SPECIFICATION

(11) **EP 3 652 310 B1**
(45) Date of publication and mention of the grant of the patent: **03.05.2023**
(21) Application number: 17917339.8
(22) Date of filing: 29.09.2017
(51) Int. Cl.: C12N 9/22, C12N 15/00, C12N 15/09, C12N 15/11, C12N 15/864, C12N 5/10

(54) **GENE EDITING SYSTEM FOR CORRECTING SPLICING DEFECTS**
GEN-EDITIERSYSTEM ZUR KORREKTUR VON SPLEISSFEHLERN
SYSTÈME D'ÉDITION DE GÈNE POUR CORRIGER LES DÉFAUTS D'ÉPISSAGE

(30) Priority: 10.07.2017 US 201762530703 P
(43) Date of publication of application: 20.05.2020
(73) Proprietor: The Hospital for Sick Children, Toronto, ON M5G 1X8 (CA)
(72) Inventor: COHN, Ronald D., Toronto Ontario M3H 3V8 (CA); KEMALADEWI, Dwi Utami, Pennsylvania 15201 (US); IVAKINE, Evgueni, Burlington Ontario L7L 3J5 (CA)
(74) Representative: Appleyard Lees IP LLP
(86) International application number: PCT/CA2017/051152
(87) International publication number: WO 2019/010559

(56) References cited:
- WO-A1-2015/138510
- HAO YIN ET AL: "Therapeutic genome editing by combined viral and non-viral delivery of CRISPR system components in vivo", NATURE BIOTECHNOLOGY, vol. 34, no. 3, 1 February 2016 (2016-02-01), pages 328-333, XP055569762, us ISSN: 1087-0156, DOI: 10.1038/nbt.3471
- F. ANN RAN ET AL: "In vivo genome editing using Staphylococcus aureus Cas9", HHS PUBLIC ACCESS AUTHOR MANUSCRIPT, vol. 520, no. 7546, 1 April 2015 (2015-04-01), pages 1-28, XP055485643, DOI: 10.1038/nature14299 Retrieved from the Internet: URL:https://www.ncbi.nlm.nih.gov/pmc/artic les/PMC4393360/>
- DWI U KEMALADEWI ET AL: "Correction of a splicing defect in a mouse model of congenital muscular dystrophy type 1A using a homology-directed-repair-independent mechanism", NATURE MEDICINE, vol. 23, no. 8, 17 July 2017 (2017-07-17), pages 984-989, XP055465597, New York ISSN: 1078-8956, DOI: 10.1038/nm.4367
- KEMALADEWI DWI U ET AL: "A mutation-independent approach for muscular dystrophy via upregulation of a modifier gene", NATURE, MACMILLAN JOURNALS LTD, LONDON, vol. 572, no. 7767, 24 July 2019 (2019-07-24), pages 125-130, XP036848585, ISSN: 0028-0836, DOI: 10.1038/S41586-019-1430-X [retrieved on 2019-07-24]
- KEMALADEWI DU et al.: "Exon Snipping in Duchenne Muscular Dystrophy", Trends Mol Med., vol. 22, no. 3, March 2016 (2016-03), pages 187-189, XP029434246,
- RUAN GX et al.: "CRISPRICas9-Mediated Genome Editing as a Therapeutic Approach for Leber Congenital Amaurosis 10", Mol Ther., vol. 25, no. 2, 1 February 2017 (2017-02-01), pages 331-341, XP055514315,
- YIN H et al.: "Therapeutic genome editing by combined viral and non-viral delivery of CRISPR system components in vivo", Nat Biotechnol, vol. 34, no. 3, March 2016 (2016-03), pages 328-333, XP055569762,
- YANG Y et al.: "A dual AAV system enables the Cas9-mediated correction of a metabolic liver disease in newborn mice", Nat Biotechnol, vol. 34, no. 3, March 2016 (2016-03), pages 334-338, XP055569763,
- OSBORN MJ et al.: "Fanconi anemia gene editing by the CRISPR/Cas9 system", Hum Gene Ther, vol. 26, no. 2, February 2015 (2015-02), pages 114-126, XP055283926,
- LONG C et al.: "Prevention of muscular dystrophy in mice by CRISPRICas9-mediated editing of germline DNA", Science, vol. 345, no. 6201, 5 September 2014 (2014-09-05), pages 1184-1188, XP055159130,

## Description

### FIELD OF THE DISCLOSURE

The present description relates generally to the field of gene editing. More particularly, the description relates to systems and methods for constituting functional donor splice sites.

### BACKGROUND OF THE DISCLOSURE

About 10% of -80,000 mutations reported in the human gene mutation database (HGMD) affect splice sites (1). In the HGMD, there are 3390 disease-causing mutations that occur at the +1 donor splice site. These mutations affect 2754 exons in 901 genes. The prevalence is even higher for neuromuscular disorders (NMDs) due to the unusually large size and multiexonic structure of genes encoding muscle structural proteins, further highlighting the importance of these mutations in NMDs (2). Therapeutic genome editing can be exploited to correct disease-causing mutations (3).

As an example of one clinical application, merosin-deficient congenital muscular dystrophy type 1A (MDC1A) is an autosomal recessive neuromuscular disease characterized by neonatal onset of muscle weakness, hypotonia, dysmyelinating neuropathy, and minor brain abnormalities (4). Splice site mutations are estimated to affect ~40% of the MDC1A patient population (5, 6). Causative mutations are located in the *LAMA2* gene, which encodes the α2 chain (Lama2) of laminin-211 (or merosin) heterotrimeric protein complex expressed in the basement membrane of muscle and Schwann cells. In MDC1A, laminin-211 loses its proper interactions with receptors such as integrin α7β1 and dystroglycan, resulting in muscle and Schwann cells apoptosis and degeneration, which leads to fibrosis and loss of muscle function.

So far, development of therapeutic strategies for MDC1A have been mainly focused on preventing fibrosis and apoptosis (4). The degree of LAMA2 deficiency highly correlates with the clinical severity in patients and mouse models (5). The lack of a functional Lama2 leads to the development of severe muscle atrophy and hind limb paralysis in mice. Therefore, restoration of LAMA2 expression holds a tremendous potential for the treatment of MDC1A. It has previously been demonstrated that muscle-specific overexpression of Laminin-211 in merosin-deficient mice improved muscle pathology, but not the associated paralysis, indicating that correction of the peripheral neuropathy requires restoration of Lama2 beyond skeletal muscles (7).

Previously (8-10), the correction of point mutations, e.g. splice site mutations, has mainly been accomplished via the homology-directed repair (HDR) pathway, which is extremely inefficient in post-mitotic tissues such as skeletal muscles, hampering its therapeutic utility in NMDs (11). A standard gene therapy approach to reintroduce the *LAMA2* coding region into the genome is impeded by its large size. Moreover, a strategy to miniaturize the gene, as in the case of microdystrophin or exon skipping in Duchenne muscular dystrophy, is unlikely to be successful due to the lack of redundant regions within the protein (12).

The ability to correct disease-causing mutations has been improved by the discovery and development of the CRISPR/Cas9 genome editing technology (3, 9). Cas9 endonuclease generates double stranded-break (DSB) in a specific genomic region, which is located adjacent to a protospacer-adjacent motif (PAM) and targeted by a complementary single guide RNA (sgRNA). In the presence of a DNA repair template, a precise genomic modification can take place through homology-directed repair (HDR), whereas non-homologous end-joining (NHEJ) repairs Cas9-induced breaks in the absence of the exogenous template. Moreover, two DSBs can be simultaneously introduced to generate genomic deletions (10-12). The discovery of Cas9 proteins derived from various species which differ in their PAM sequence requirements (3, 13, 35, 36), as well as engineered Cas9 derivatives with purposefully altered PAM specificities (37, 38) offer unprecedented flexibility in genome editing endeavours.

In the context of metabolic liver diseases, injection of CRISPR/Cas9 and a DNA repair template has been shown to correct *Fah* and *Otc* mutations in vivo (13-15). However, HDR in post-mitotic tissues, such as skeletal muscle and/or neurons is extremely inefficient.

It is an object of the present disclosure to mitigate and/or obviate one or more of the above deficiencies.

### SUMMARY OF THE DISCLOSURE

The present invention is defined in the appended claims. The present disclosure aids in the understanding of the invention.

In an aspect, a genome editing system is provided. The system comprises at least one nuclease that generates blunt-ended DNA breaks in a sequence-specific manner, wherein the genome editing system is configured to form a first and a second blunt-ended double strand break in an intron of a gene, wherein the intron comprises a splice donor site mutation that alters splice site recognition, thereby excising a segment of the intron and simultaneously joining DNA ends flanking the excised segment of the intron to constitute a functional donor splice site.

In an aspect of the system, the excising of the segment of the intron and simultaneously joining the DNA ends flanking the excised segment of the intron to constitute a functional donor splice site results in restored splice site recognition in the intron.

In an aspect of the system, the joining of the DNA ends flanking the excised segment of the intron to constitute a functional donor splice site occurs through a homology directed repair-independent mechanism.

In an aspect of the system, the joining of the DNA ends flanking the excised segment of the intron to constitute a functional donor splice site occurs through non-homologous end joining.

In an aspect of the system, the excising occurs at a mutation-eliminating cut site of the intron and at a splice site-generating cut site of the intron of the gene. In an aspect, the mutation-eliminating cut site and/or the splice generating cut site is at a Cas9 cleavage site occurring in the presence of a PAM nucleotide sequence that is: NGG, NAG, NGA, NGCG, NNGRRT, NNNRRT, NNAGAA, NNNNRYAC, or YG. In an aspect, the splice site-generating cut site is positioned before a branch splice point for an adjacent exon. In an aspect, the splice site-generating cut site is positioned at least 25 base pairs from the adjacent exon.

In an aspect of the system, there is at least one nuclease that generates blunt-ended DNA breaks, which is a Cas9 polypeptide that recognizes a Protospacer Adjacent Motif (PAM) and wherein the system further comprises a mutation-eliminating single guide RNA (sgRNA) and a splice site-generating sgRNA. In an aspect, the PAM has the nucleotide sequence NNGRRT and the mutation-eliminating cut site of the intron is positioned 3 nucleotides in the 5' direction of the end of the PAM. In an aspect, the mutation-eliminating sgRNA and the splice site-generating sgRNA each comprise a CRISPR RNA (crRNA) portion that is 17 to 27 nucleotides in length. In an aspect, the mutation-eliminating sgRNA comprises the nucleotide sequence aaaacatatatataatacatatGGT (SEQ ID NO: 1).

In an aspect of the system, there is at least one nuclease that generates blunt-ended DNA breaks, which is a fusion polypeptide comprising a catalytically inactive Cas9 polypeptide that recognizes a PAM and a nuclease that generates blunt-ended DNA breaks. In an aspect, the polypeptide further comprises a linker between the catalytically inactive Cas9 and the nuclease. In an aspect, the size of the linker determines the location of the blunt-ended DNA breaks.

In an aspect of the system, there is at least one nuclease that generates blunt-ended DNA breaks, which is a fusion polypeptide comprising a DNA recognizing protein domain and a nuclease that generates blunt-ended DNA breaks. In an aspect, the DNA recognizing protein domain comprises a zinc finger, and wherein the system comprises a pair of fusion polypeptides, each comprising a zinc finger and a nuclease that upon dimerization of the nuclease domains generates blunt-ended DNA breaks. In an alternative aspect, the DNA recognizing protein domain comprises a TALE polypeptide, and wherein the system comprises a pair of fusion polypeptides, each comprising a TALE polypeptide and a nuclease that upon dimerization of the nuclease domains generates blunt-ended DNA breaks. In an aspect, the nuclease is Mlyl. In an aspect, the polypeptide further comprises a linker between the DNA recognizing protein domain and the nuclease that generates blunt-ended DNA breaks. In an aspect, the size of the linker determines the location of the blunt-ended DNA breaks.

In an aspect of the system, the mutation occurs at the +1 position of the splice donor site. In various aspects, the mutation occurs at the +2, +3, +4, +5, or +6 position of the splice donor site.

In an aspect of the system, the system further comprises a delivery vehicle for delivery of the at least one nuclease that generates blunt-ended DNA breaks in a sequence-specific manner.

In an aspect of the system, polynucleotides coding for at least one nuclease that generates blunt-ended DNA breaks in a sequence-specific manner is in a vector. In an aspect, the nuclease is Cas9 and the vector further comprises a mutation-eliminating single sgRNA or a splice site-generating sgRNA. In an aspect, the delivery vehicle is an adeno-associated virus (AAV) vector. In various aspects, the delivery vehicle is an AAV2, AAV3, AAV4, AAV5, AAV6, AAV7, AAV8 or AAV9 vector. In an aspect, the delivery vehicle is an AAV9 vector.

In an aspect of the system, the delivery vehicle is a liposome or a nanoparticle.

In an aspect of the system, the mutation is a pathogenic mutation.

In an aspect of the system, the gene is a gene listed in Table 1.

In an aspect of the system, the gene is a *Lama2* gene. In an aspect, the mutation is a c.417+1 g→a mutation. In an aspect, the mutation is in a subject having merosin deficient congenital muscular dystrophy (MDC1A).

In an aspect, the system as described herein is for use in the treatment of merosin deficient congenital muscular dystrophy (MDC1A) in a subject.

In an aspect, a composition is provided. The composition comprises at least one nuclease that generates blunt-ended DNA breaks in a sequence-specific manner, wherein the composition is configured to form a first and a second blunt-ended double strand break in an intron of a gene, wherein the intron comprises a splice donor site mutation that alters splice site recognition, thereby excising a segment of the intron and simultaneously joining DNA ends flanking the excised segment of the intron to constitute a functional donor splice site.

In an aspect of the composition, the excising of the segment of the intron and simultaneously joining the DNA ends flanking the excised segment of the intron to constitute a functional donor splice site results in restored splice site recognition in the intron.

In an aspect of the composition, the joining of the DNA ends flanking the excised segment of the intron to constitute a functional donor splice site occurs through a homology directed repair-independent mechanism.

In an aspect of the composition, the joining of the DNA ends flanking the excised segment of the intron to constitute a functional donor splice site occurs through non-homologous end joining.

In an aspect of the composition, the excising occurs at a mutation-eliminating cut site of the intron and at a splice site-generating cut site of the intron of the gene. In an aspect, the mutation-eliminating cut site and/or the splice generating cut site is at a Cas9 cleavage site occurring in the presence of a PAM nucleotide sequence that is: NGG, NAG, NGA, NGCG, NNGRRT, NNNRRT, NNAGAA, NNNNRYAC, or YG. In an aspect, the splice site-generating cut site is positioned before a branch splice point for an adjacent exon. In an aspect, the splice site-generating cut site is positioned at least 25 base pairs from the adjacent exon.

In an aspect of the composition, at least one nuclease that generates blunt-ended DNA breaks is a Cas9 polypeptide that recognizes a Protospacer Adjacent Motif (PAM) and wherein the system further comprises a mutation-eliminating single guide RNA (sgRNA) and a splice site-generating sgRNA. In an aspect, the PAM has the nucleotide sequence NNGRRT and the mutation-eliminating cut site of the intron is positioned 3 nucleotides in the 5' direction of the end of the PAM. In an aspect, the mutation-eliminating sgRNA and the splice site-generating sgRNA each comprise a CRISPR RNA (crRNA) portion that is 17 to 27 nucleotides in length. In an aspect, the mutation-eliminating sgRNA comprises the nucleotide sequence aaaacatatatataatacatatGGT (SEQ ID NO: 1).

In an aspect of the composition, at least one nuclease that generates blunt-ended DNA breaks is a fusion polypeptide comprising a catalytically inactive Cas9 polypeptide that recognizes a PAM and a nuclease that generates blunt-ended DNA breaks. In an aspect, the polypeptide further comprises a linker between the catalytically inactive Cas9 and the nuclease. In an aspect, the size of the linker determines the location of the blunt-ended DNA breaks.

In an aspect of the composition, at least one nuclease that generates blunt-ended DNA breaks is a fusion polypeptide comprising a DNA recognizing protein domain and a nuclease that generates blunt-ended DNA breaks. In an aspect, the DNA recognizing protein domain comprises a zinc finger, and wherein the system comprises a pair of fusion polypeptides, each comprising a zinc finger and a nuclease that upon dimerization of the nuclease domains generates blunt-ended DNA breaks. In an alternative aspect, the DNA recognizing protein domain comprises a TALE polypeptide, and wherein the system comprises a pair of fusion polypeptides, each comprising a TALE polypeptide and a nuclease that upon dimerization of the nuclease domains generates blunt-ended DNA breaks. In an aspect, the nuclease is Mlyl. In an aspect, the polypeptide further comprises a linker between the DNA recognizing protein domain and the nuclease that generates blunt-ended DNA breaks. In an aspect, the size of the linker determines the location of the blunt-ended DNA breaks.

In an aspect of the composition, the mutation occurs at the +1 position of the splice donor site. In various aspects, the mutation occurs at the +2, +3, +4, +5, or +6 position of the splice donor site.

In an aspect of the composition, the composition further comprises a delivery vehicle for delivery of the at least one nuclease that generates blunt-ended DNA breaks in a sequence-specific manner.

In an aspect of the composition, polynucleotides coding for at least one nuclease that generates blunt-ended DNA breaks in a sequence-specific manner is in a vector. In an aspect, the nuclease is Cas9 and the vector further comprises a mutation-eliminating single sgRNA or a splice site-generating sgRNA. In an aspect, the delivery vehicle is an adeno-associated virus (AAV) vector. In various aspects, the delivery vehicle is an AAV2, AAV3, AAV4, AAV5, AAV6, AAV7, AAV8 or AAV9 vector. In an aspect, the delivery vehicle is an AAV9 vector.

In an aspect of the composition, the delivery vehicle is a liposome or a nanoparticle.

In an aspect of the composition, the mutation is a pathogenic mutation.

In an aspect of the composition, the gene is a gene listed in Table 1.

In an aspect of the composition, the gene is a *Lama2* gene. In an aspect, the mutation is a c.417+1 g→a mutation. In an aspect, the mutation is in a subject having merosin deficient congenital muscular dystrophy (MDC1A).

In an aspect, the composition as described herein is for use in the treatment of merosin deficient congenital muscular dystrophy (MDC1A) in a subject.

In an aspect, a method of constituting a functional donor splice site in a gene is provided. The gene comprises a mutation in a donor splice site that alters splice site recognition, and the method comprises delivering a genome editing system to the gene, the genome editing system comprising at least one nuclease that generates blunt-ended DNA breaks in a sequence-specific manner, wherein the genome editing system is configured to form a first and a second blunt-ended double strand break in an intron of a gene, wherein the intron comprises a splice donor site mutation that alters splice site recognition, thereby excising a segment of the intron and simultaneously joining DNA ends flanking the excised segment of the intron to constitute a functional donor splice site.

In an aspect of the method, the excising of the segment of the intron and simultaneously joining the DNA ends flanking the excised segment of the intron to constitute a functional donor splice site results in restored splice site recognition in the intron.

In an aspect of the method, the joining of the DNA ends flanking the excised segment of the intron to constitute a functional donor splice site occurs through a homology directed repair-independent mechanism.

In an aspect of the method, the joining of the DNA ends flanking the excised segment of the intron to constitute a functional donor splice site occurs through non-homologous end joining.

In an aspect of the method, the excising occurs at a mutation-eliminating cut site of the intron and at a splice site-generating cut site of the intron of the gene. In an aspect, the mutation-eliminating cut site and/or the splice generating cut site is at a Cas9 cleavage site occurring in the presence of a PAM nucleotide sequence that is: NGG, NAG, NGA, NGCG, NNGRRT, NNNRRT, NNAGAA, NNNNRYAC, or YG. In an aspect, the splice site-generating cut site is positioned before a branch splice point for an adjacent exon. In an aspect, the splice site-generating cut site is positioned at least 25 base pairs from the adjacent exon.

In an aspect of the method, at least one nuclease that generates blunt-ended DNA breaks is a Cas9 polypeptide that recognizes a Protospacer Adjacent Motif (PAM) and wherein the system further comprises a mutation-eliminating single guide RNA (sgRNA) and a splice site-generating sgRNA. In an aspect, the PAM has the nucleotide sequence NNGRRT and the mutation-eliminating cut site of the intron is positioned 3 nucleotides in the 5' direction of the end of the PAM. In an aspect, the mutation-eliminating sgRNA and the splice site-generating sgRNA each comprise a CRISPR RNA (crRNA) portion that is 17 to 27 nucleotides in length. In an aspect, the mutation-eliminating sgRNA comprises the nucleotide sequence aaaacatatatataatacatatGGT (SEQ ID NO: 1).

In an aspect of the method, at least one nuclease that generates blunt-ended DNA breaks is a fusion polypeptide comprising a catalytically inactive Cas9 polypeptide that recognizes a PAM and a nuclease that generates blunt-ended DNA breaks. In an aspect, the polypeptide further comprises a linker between the catalytically inactive Cas9 and the nuclease. In an aspect, the size of the linker determines the location of the blunt-ended DNA breaks.

In an aspect of the method, at least one nuclease that generates blunt-ended DNA breaks is a fusion polypeptide comprising a DNA recognizing protein domain and a nuclease that generates blunt-ended DNA breaks. In an aspect, the DNA recognizing protein domain comprises a zinc finger, and wherein the system comprises a pair of fusion polypeptides, each comprising a zinc finger and a nuclease that upon dimerization of the nuclease domains generates blunt-ended DNA breaks. In an alternative aspect, the DNA recognizing protein domain comprises a TALE polypeptide, and wherein the system comprises a pair of fusion polypeptides, each comprising a TALE polypeptide and a nuclease that upon dimerization of the nuclease domains generates blunt-ended DNA breaks. In an aspect, the nuclease is Mlyl. In an aspect, the polypeptide further comprises a linker between the DNA recognizing protein domain and the nuclease that generates blunt-ended DNA breaks. In an aspect, the size of the linker determines the location of the blunt-ended DNA breaks.

In an aspect of the method, the mutation occurs at the +1 position of the splice donor site. In various aspects, the mutation occurs at the +2, +3, +4, +5, or +6 position of the splice donor site.

In an aspect of the method, the system further comprises a delivery vehicle for delivery of the at least one nuclease that generates blunt-ended DNA breaks in a sequence-specific manner.

In an aspect of the method, polynucleotides coding for the at least one nuclease that generates blunt-ended DNA breaks in a sequence-specific manner is in a vector. In an aspect, the nuclease is Cas9 and the vector further comprises a mutation-eliminating single sgRNA or a splice site-generating sgRNA. In an aspect, the delivery vehicle is an adeno-associated virus (AAV) vector. In various aspects, the delivery vehicle is an AAV2, AAV3, AAV4, AAV5, AAV6, AAV7, AAV8 or AAV9 vector. In an aspect, the delivery vehicle is an AAV9 vector.

In an aspect of the method, the delivery vehicle is a liposome or a nanoparticle.

In an aspect of the method, the mutation is a pathogenic mutation.

In an aspect of the method, the gene is a gene listed in Table 1.

In an aspect of the method, the gene is a *Lama2* gene. In an aspect, the mutation is a c.417+1 g→a mutation. In an aspect, the mutation is in a subject having merosin deficient congenital muscular dystrophy (MDC1A).

In an aspect, the method as described herein is for use in the treatment of merosin deficient congenital muscular dystrophy (MDC1A) in a subject.

In an aspect, an isolated cell comprising a gene whose mutation at a splice donor site has been repaired according to any of the methods as described herein is provided. In an aspect, the cell is a postmitotic cell. In an aspect, the cell is a myocyte, a neuron or a cardiomyocyte.

The details of one or more aspects are set forth in the accompanying drawings and description below.

### DESCRIPTION OF THE DRAWINGS

These and other features of the disclosure will become more apparent in the following detailed description in which reference is made to the appended drawings wherein:

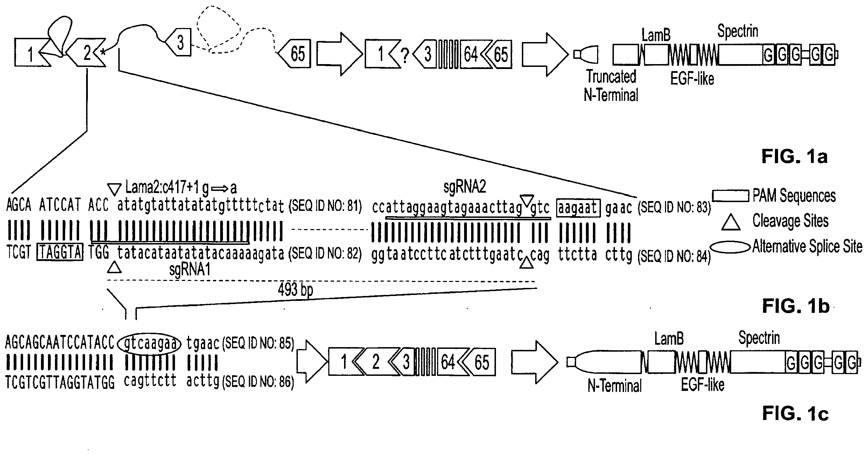

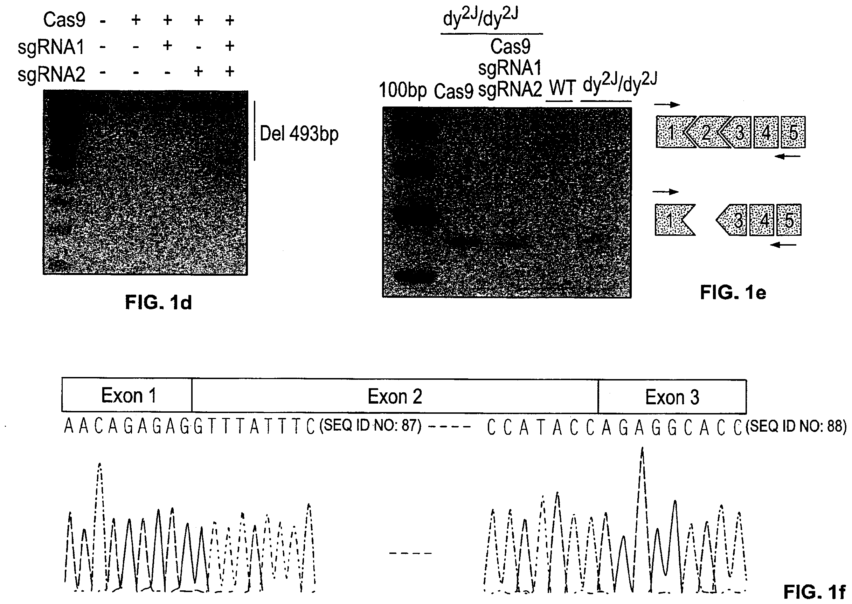

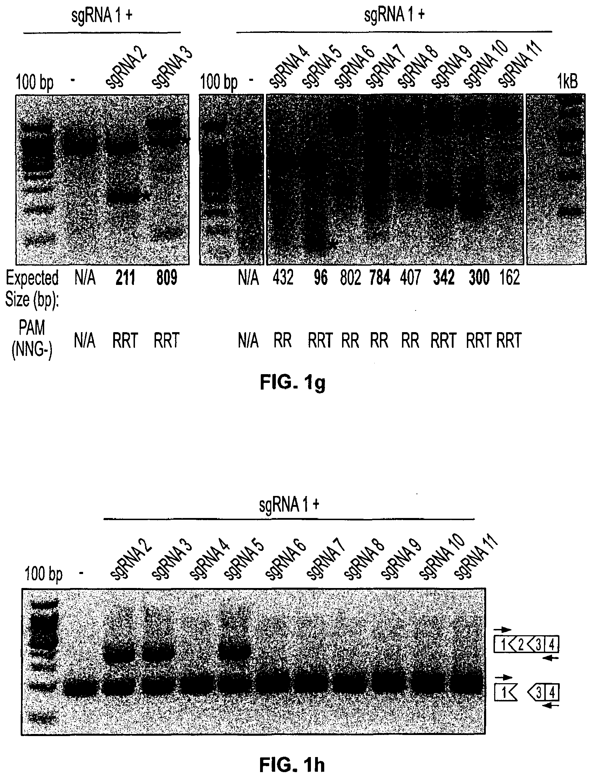

FIGs. 1A-1H are depictions of a strategy for correction of a splicing defect in the *Lama2* gene using an HDR-independent strategy. The strategy was implemented on a *dy^{2J}*/*dy^{2J}* mouse model (Fig. 1A). A SaCas9 PAM sequence near to the relevant mutation was identified and designated as sgRNA1, a "mutation-elimination sgRNA". (Fig. 1B). Nucleotides proximate to intron 2 were evaluated for potential donor splice sites (Fig. 1C). Possible "splice site-generating sgRNAs" (sgRNA2-sgRNA11) were tested in *dy^{2J}*/*dy^{2J}* myoblasts and RT-PCR followed by gel electrophoresis analysis revealed the presence of an amplicon similar to the wild-type control for sgRNA2, sgRNA3 and sgRNA5, indicating an inclusion of the exon 2 of *Lama2* after generation of intronic deletions (Figs. 1D, 1G), where asterisks in Fig. 1G indicate sgRNA combinations that produced correct deletions. RT-PCR on RNA isolated from *dy^{2J}*/*dy^{2J}* -derived myoblasts transfected with Sa Cas9 and the corresponding sgRNAs followed by gel electrophoresis analysis, where arrows correspond to primers in exons 1 and 5 (Fig. 1E) or in exons 1 and 4 (Fig. 1H) used in RT-PCR. Sanger sequencing confirmed that full-length exon 2 was successfully incorporated in the *Lama2* mRNA in sgRNA1+sgRNA2-treated *dy^{2J}*/*dy^{2J}* myoblasts (Fig. 1F).

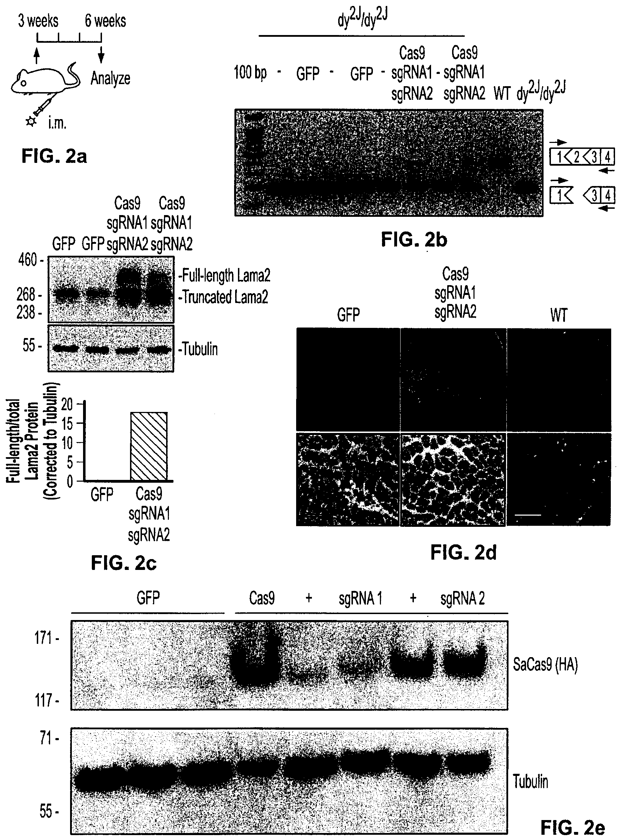

FIGs. 2A-2E are depictions of the restoration of full-length Lama2 and improvement of muscle pathology following intramuscular AAV-CRISPR administration in adult *dy^{2J}*/*dy^{2J}* mice. Delivery of AAV9-Cas9-sgRNA and AAV-Cas9-sgRNA (7.5 × 10¹¹ viral genome each by injection into the right tibialis anterior (TA) muscles of 3-week old *dy^{2J}*/*dy^{2,J}* mice, with euthanization and analysis following three weeks later (Fig. 2A). Gel electrophoresis showing expression of Cas9 following intraperitoneal administration of AAV-CRISPR in *dy^{2J}*/*dy^{2J}* mice (Fig. 2E). RT-PCR followed by gel electrophoresis analysis, with arrows depicting the location of primers used, and minus (-) signs indicating non-injected, contralateral TA muscles (Fig. 2B) and western blot, with tubulin serving as a loading control (Fig. 2C) show transcripts with inclusion of exon 2, successfully translated into full-length Lama2 protein. 8-µm cross-sections analyzed for localization of Lama2 and general muscle architecture by immunofluorescence (upper panels) and H&E (lower panels) staining, respectively (scale bar: 50 µm) (Fig. 2D).

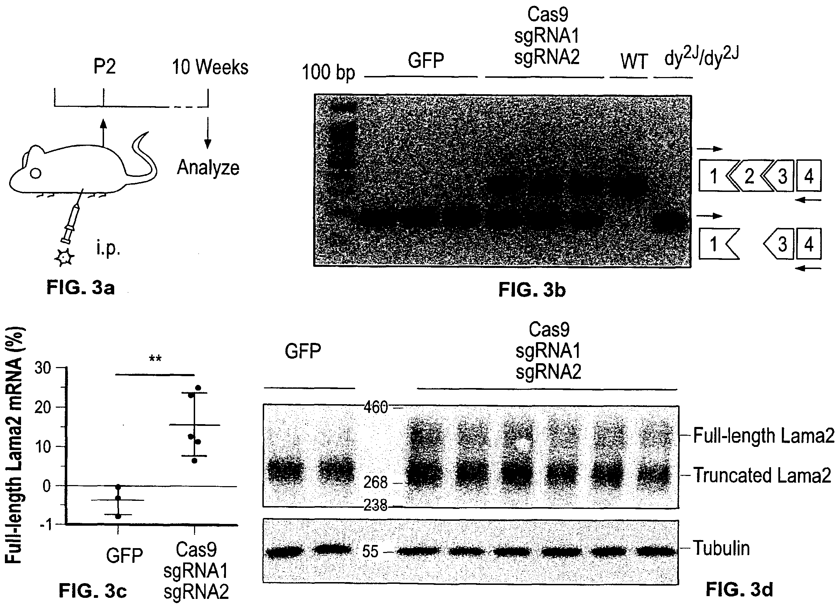

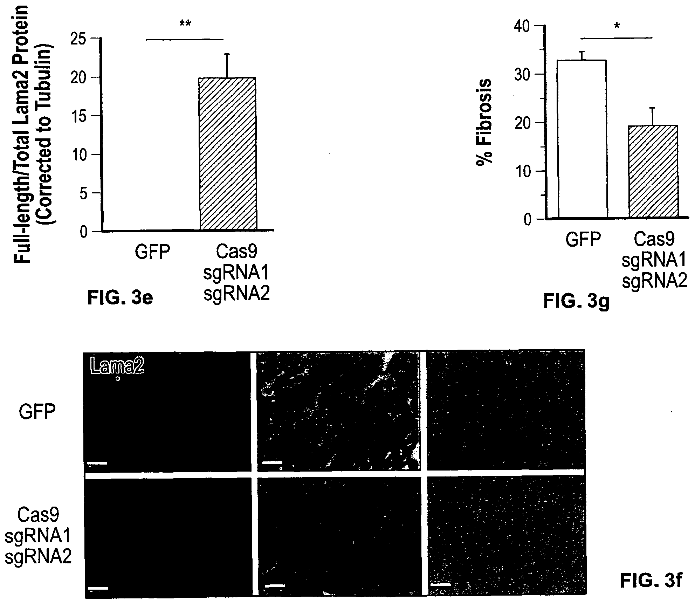

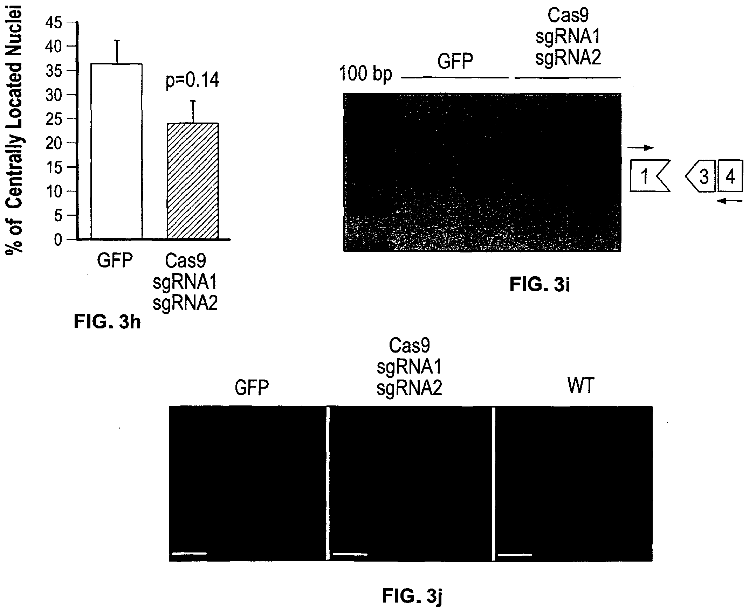

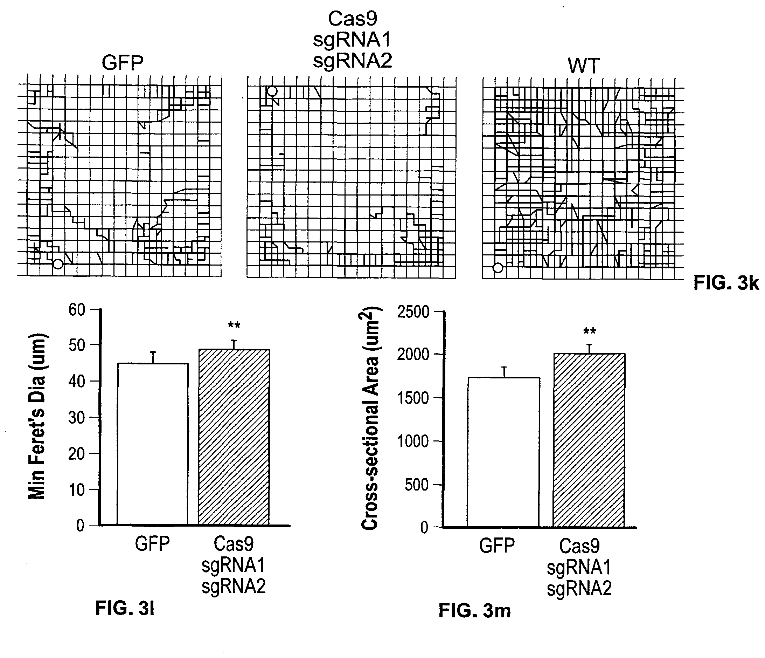

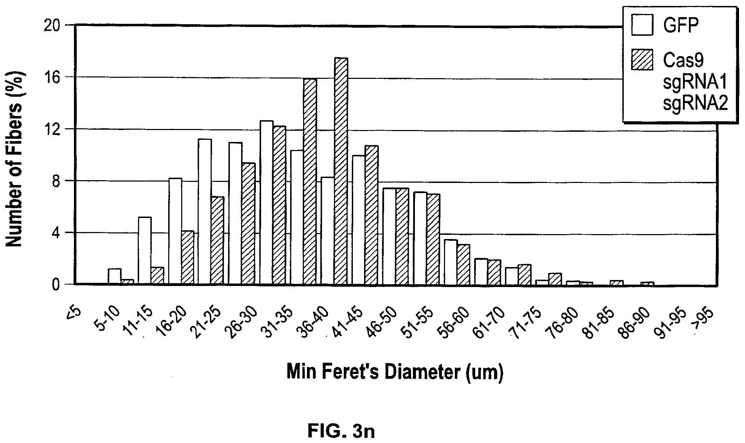

FIGs. 3A-3N are depictions of intraperitoneal administration of AAV9-CRISPR in neonatal *dy^{2J}*/*dy^{2J}* mice and the results. Two days old *dy^{2J}*/*dy^{2J}* pups were injected with AAV9-GFP (n=4) or combinations of AAV9-Cas9-sgRNA1 and AAV9Cas9-sgRNA2 (n=6) (7.5 × 10¹¹ viral genome/vector) intraperitoneally and sacrificed 10 weeks post-injection (Fig. 3A). Gastrocnemius muscle was analyzed for exon 2 inclusion by RT-PCR and gel electrophoresis, with arrows depicting the location of primers used for the RT-PCR (Fig. 3B) (three representative mice per group are shown). Graphical representation of the quantification of transcripts containing exon 2 by quantitative PCR (Fig. 3C) (presented as mean ± standard deviation). Western blot analysis of sample lysates to detect restoration of full-length Lama2 expression, with tubulin serving as a loading control (Fig. 3D). Graphical representation of densitometry analysis to quantify the amount of full-length form over total Lama2 proteins, corrected to tubulin expression (Fig. 3E) (presented as mean ± standard deviation). Images of immunofluorescence staining on 8µm gastrocnemius muscle cross-section with antibodies against Lama (Fig. 3F, left), H&E and picrosirius red staining (Fig. 3F, middle and right) (scale bar: 50 µm). Graphical depiction of the quantitation of fibrosis from Sirius red staining (Fig. 3G) (presented as mean ± SEM on n=3-5 mice/group). Graphical depiction of the percentages of myofibers with centrally located nuclei (Fig. 3H) (presented as mean ± SEM on n=3-5 mice/group). Graphical representation of morphometric analyses on gastrocnemius muscles following intraperitoneal administration in neonatal *dy^{2J}*/*dy^{2J}* mice, including assessment of mean minimum Feret's diameter (Fig 3L), average of cross-sectional area (Fig. 3M) and distribution of fiber size of gastrocnemius muscles (Fig. 3N) isolated 10 weeks post injection. RT-PCR and gel electrophoresis analysis of RNA isolated from sciatic nerves (Fig. 3l). Images of immunofluorescence staining of sciatic nerves to detect Lama2 expression (Fig. 3J). Plot of examples of mouse trajectories in 20-minutes open field test (Fig. 3K). Statistical analyses were performed using Student's t-test. *p<0.05. **p<0.01.

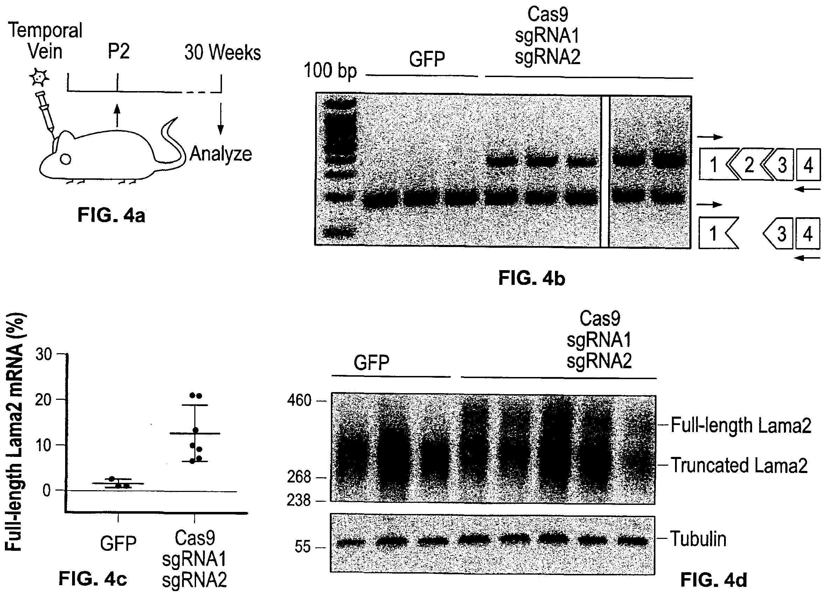

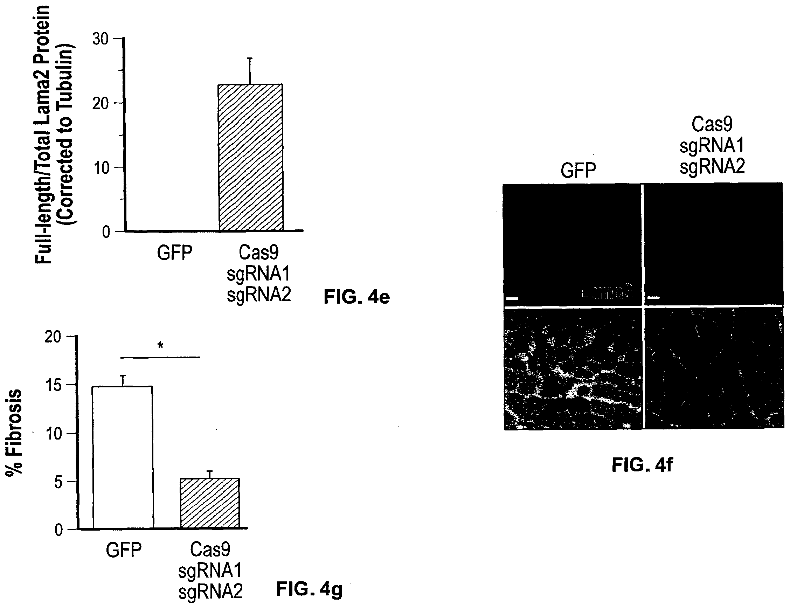

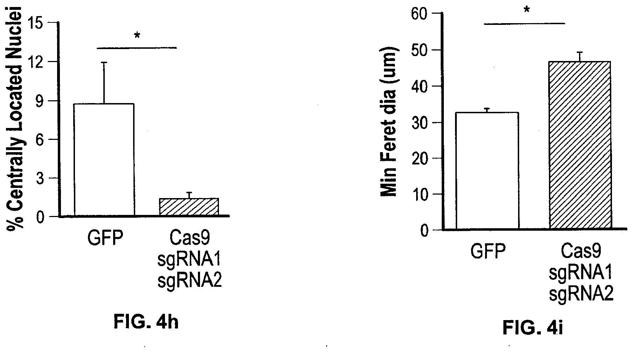

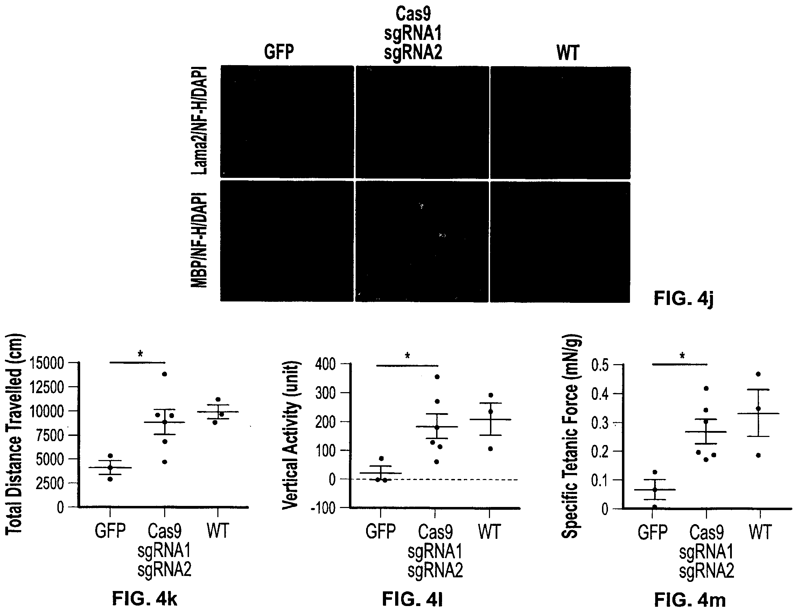

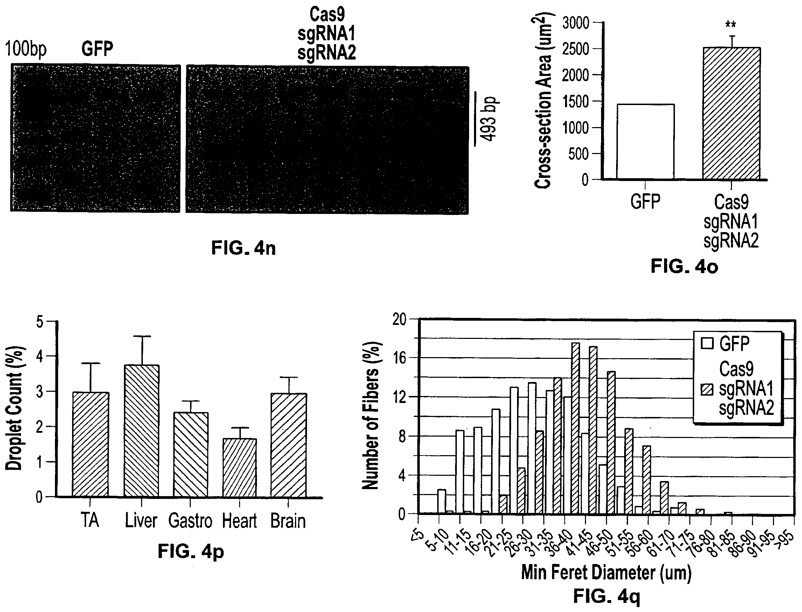

FIGs. 4A-4Q are depictions of temporal vein administration of AAV9-CRISPR in neonatal *dy^{2J}*/*dy^{2J}* mice and the results. Two days old *dy^{2J}*/*dy^{2J}* pups were injected with AAV9-GFP (n=3) or combinations of AAV9-Cas9-sgRNA1 and AAV9Cas9-sgRNA2 (n=7) (7.5 × 10¹¹ viral genome/vector) via temporal vein route and sacrificed 30 weeks post-injection (Fig. 4A). PCR and gel electrophoresis on genomic DNA isolated from TA muscles to detect Cas9-mediated deletions (Fig. 4N), with muscles isolated from mice treated with AAV-CRISPR showing -493 bp deletions. Graphical representation of digital droplet PCR amplification, showing the percentage of edited DNA and plotted as average ± SEM of n=3-6 mice /group (Fig. 4P). TA muscle was analyzed for exon 2 inclusion by RT-PCR and gel electrophoresis, with arrows depicting the location of primers used for the RT-PCR (Fig. 4B). Graphical representation of the quantification of transcripts containing exon 2 by quantitative PCR (Fig. 4C) (presented as mean ± standard deviation). Western blot analysis of sample lysates to detect restoration of full-length Lama2 expression, with tubulin serving as a loading control (Fig. 4D). Graphical representation of densitometry analysis to quantify the amount of full-length form over total Lama2 proteins, corrected to tubulin expression (Fig. 4E) (presented as mean ± standard deviation). Images of immunofluorescence staining on 8 µm TA muscle cross-section with antibodies against Lama (Fig. 4F, upper panels) and H&E staining (Fig. 4F, lower panels) (scale bar: 50 µm). Graphical depiction of the quantitation of fibrosis from H&E staining of the TA muscle cross-section shown in Fig. 4F (Fig. 4G) (presented as mean ± SEM on n=3-7 mice/group). Graphical depiction of the percentages of myofibers with centrally located nuclei (Fig. 4H) (presented as mean ± SEM on n=3-7 mice/group). Graphical representation of morphometric analyses on TA muscles following temporal vein administration in neonatal *dy^{2J}*/*dy^{2J}* mice, including assessment of mean minimum Feret's diameter (Fig 4I), cross-sectional area plotted as average ± SEM of n=3-6 mice/group (Fig. 40) and distribution of fiber size measured as minimum Feret's diameter, of TA muscles (Fig. 4Q) isolated 30 weeks post injection. Images of immunofluorescence staining of sciatic nerves to detect Lama2 expression (green upper panel) or myelin binding protein (MBP, green lower panel) and neurofilament H (red), with DAPI (blue) staining the nucleus (Fig. 4J). Graphical representations of assessment of mice tested in open field chamber, where total distance (Fig. 4K) and vertical activity (Fig. 4L were assessed. Graphical representation of specific tetanic force measured using in vivo muscle function analyzer (Fig. 4M). Statistical analyses were performed using Student's t-test. *p<0.05. **p<0.01.

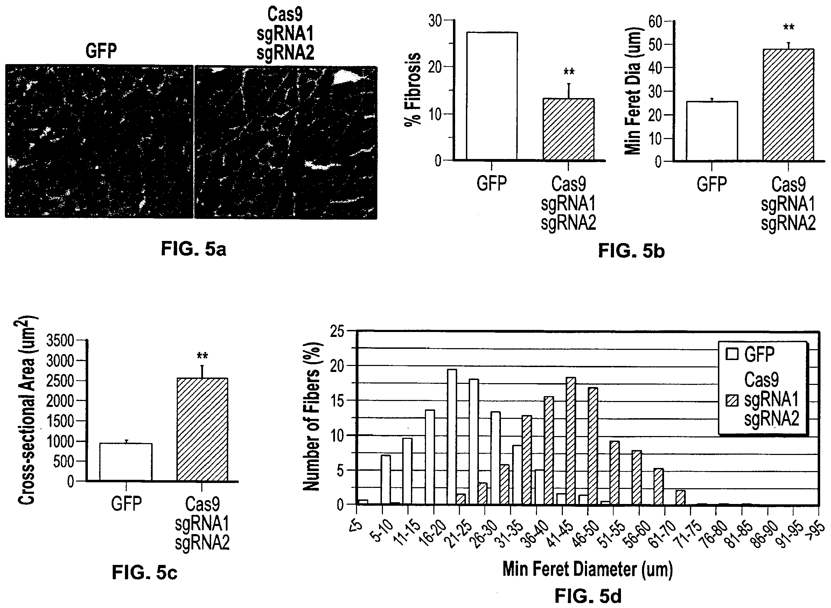

FIGs. 5A-5D depict analyses of gastrocnemius muscles following temporal vein administration of AAV9-CRISPR. H&E-stained muscle cross sections of mice administered AAV9-GFP or combination of AAV9-Cas9-sgRNA1 and AAV-Cas9-sgRNA2 (Fig. 5A). Graphical representation of % fibrosis (Fig. 5B, left panel) and minimum Feret's diameter (Fig. 5B, right panel) in cross sections shown in Fig. 5A. Graphical representation of cross sectional area in cross sections shown in Fig. 5A (Fig. 5C). Graphical representation of distribution of fiber size in cross sections shown in Fig. 5A (Fig. 5D). Data in Figs. 5B and 5C were plotted as average ± SEM of n=3-6 mice/group. Statistical analysis was performed using Student's t-test. **p<0.01.

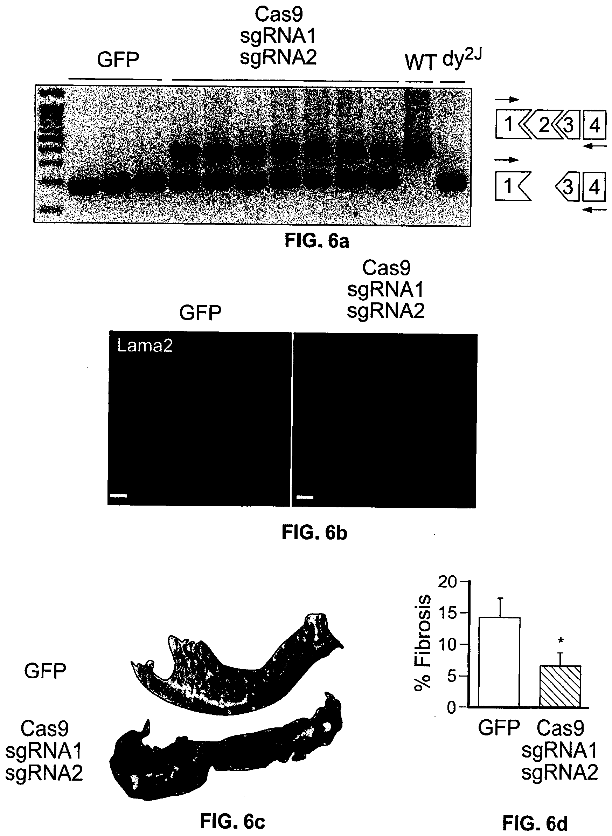

FIGs. 6A-6D depict analyses of diaphragm muscles following temporal vein administration of AAV9-CRISPR. Diaphragm muscle from mice administered AAV9-GFP or combination of AAV9-Cas9-sgRNA1 and AAV-Cas9-sgRNA2 was analyzed for exon 2 inclusion by RT-PCR and gel electrophoresis, with arrows depicting the location of primers used for the RT-PCR (Fig. 6A). Images of immunofluorescence staining of diaphragm muscles to detect Lama2 expression in mice administered AAV9-GFP or combination of AAV9-Cas9-sgRNA1 and AAV-Cas9-sgRNA2 (Fig. 6B). H&E-stained muscle cross sections of mice administered AAV9-GFP or combination of AAV9-Cas9-sgRNA1 and AAV-Cas9-sgRNA2 (Fig. 6C). Graphical representation of % fibrosis in muscle cross sections of mice administered AAV9-GFP or combination of AAV9-Cas9-sgRNA1 and AAV-Cas9-sgRNA2 (Fig. 6D), plotted as average ± SEM of n=3-6 mice/group. Statistical analysis was performed using Student's t-test. *p<0.05.

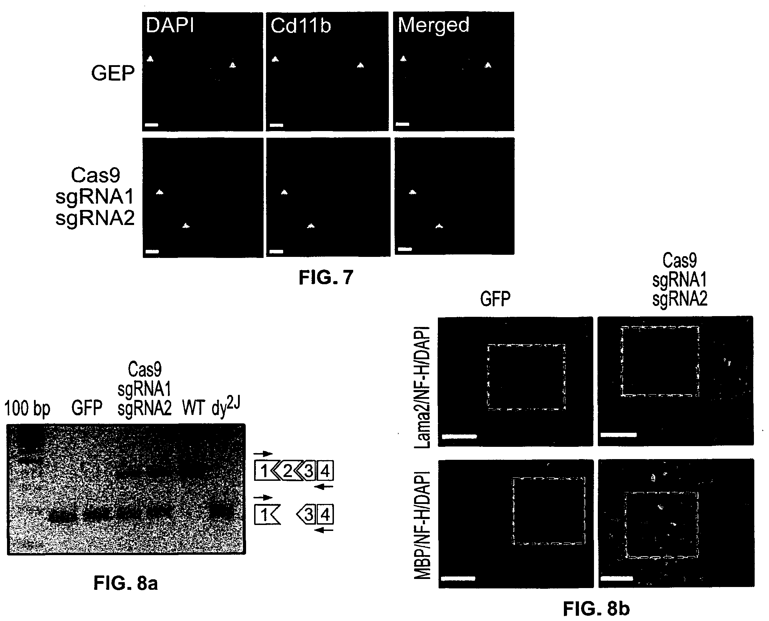

FIG. 7 shows images of inflammatory cells in TA muscles of *dy^{2J}*/*dy^{2J}* mice administered AAV9-GFP or combination of AAV9-Cas9-sgRNA1 and AAV-Cas9-sgRNA2, stained with DAPI (left panels), Cd11b (middle panels) and merged (right panels).

FIGs. 8A-8B depict analyses of sciatic nerves following temporal vein administration of AAV-CRISPR. Sciatic nerves from mice administered AAV9-GFP or combination of AAV9-Cas9-sgRNA1 and AAV-Cas9-sgRNA2 was analyzed for exon 2 inclusion by RT-PCR and gel electrophoresis, with arrows depicting the location of primers used for the RT-PCR (Fig. 8A). Images of lower magnification images of the right and middle panels of Fig. 4J (Fig. 8B).

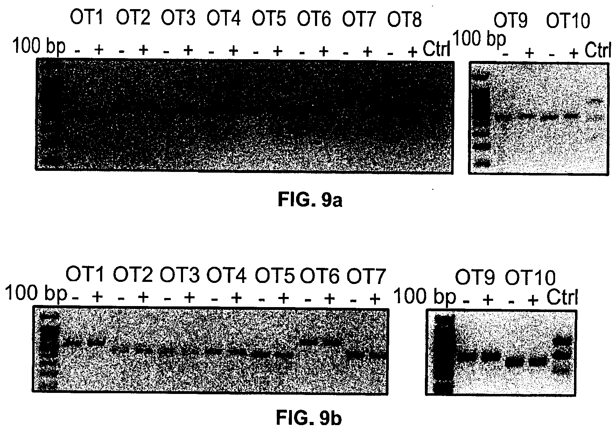

FIGs. 9A-9B depict images of off-target analyses of sgRNA1 and sgRNA2. T7E1 digestion on top ten theoretical off-target sites (OT1-OT10) of sgRNA1 (Fig. 9A) or sgRNA2 (Fig. 9B). "-" denotes the genomic DNA from mice injected with AAV9-GFP and "+" represents those injected with combination of AAV9-Cas9-sgRNA1 and AAV9-Cas9-sgRNA2.

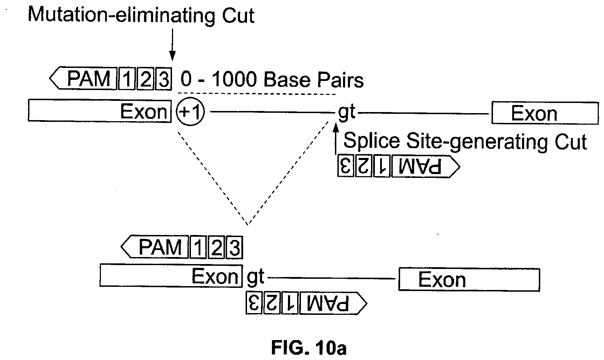

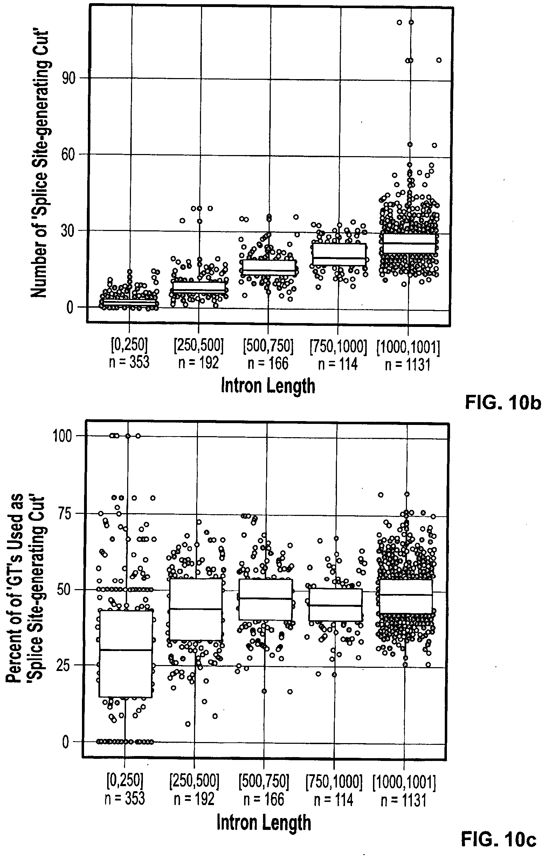

FIGs. 10A-10C depict in silico analysis of applicability of exon exclusion strategy to correct disease-causing splice site mutations. Strategy for identifying targetable +1 donor site mutations (Fig. 10A); note that while there are two possible directions of the PAM at each cut site, only one direction per cut site is shown for illustrative purposes. Absolute quantification of "splice site-generating cut" found within analyzed intron of different length (Fig. 10B). Percentage of "splice site-generating cut" sites over absolute quantification in Fig. 10B found in introns analyzed, with the X-axis showing the length of introns analyzed (Fig. 10C) ([0,250] defines introns that are shorter than 250 bp; [250,500] represents introns that are longer than or equal to 250 bp and shorter than 500 bp; [500,750] represents introns that are longer than or equal to 500 bp and shorter than 7500 bp; [750,1000] represents introns that are longer than or equal to 750 bp and shorter than 1000 bp; [1000,1001] represents introns that are longer than 1000 bp, but only the first 1000 bp were analyzed).

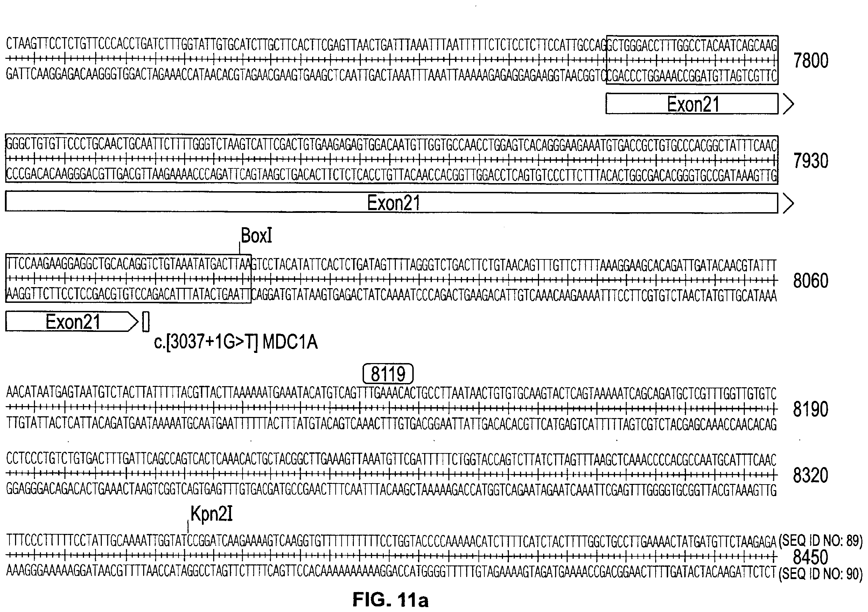

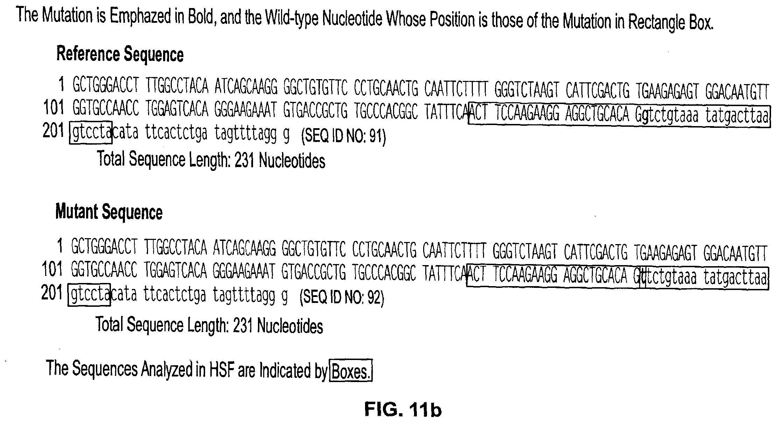

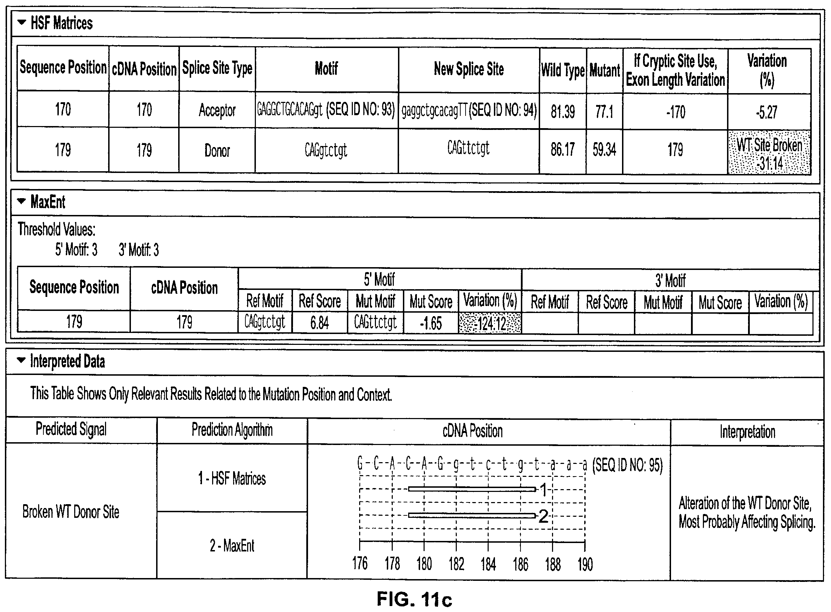

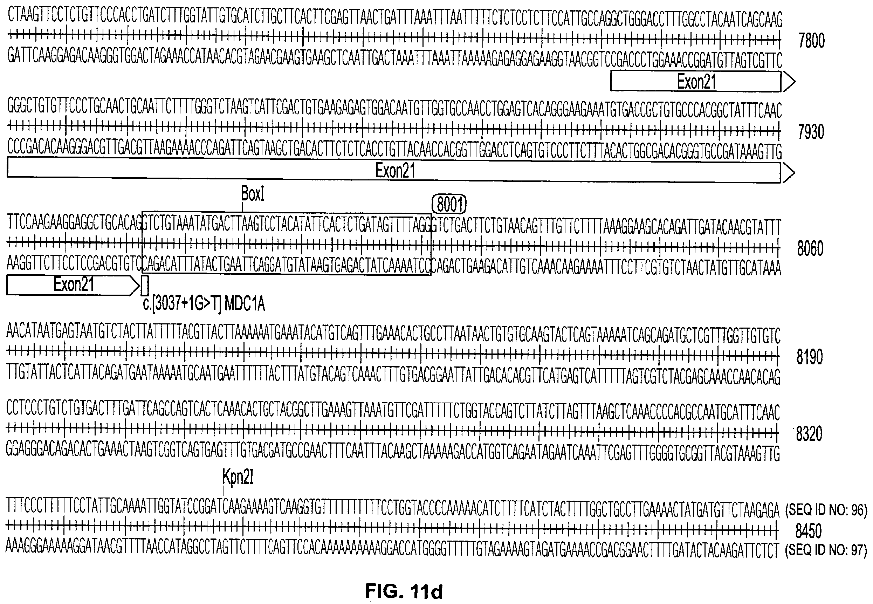

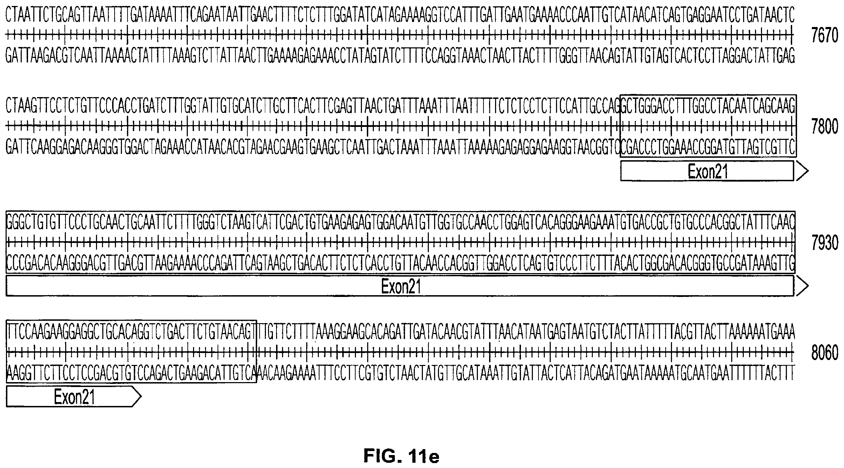

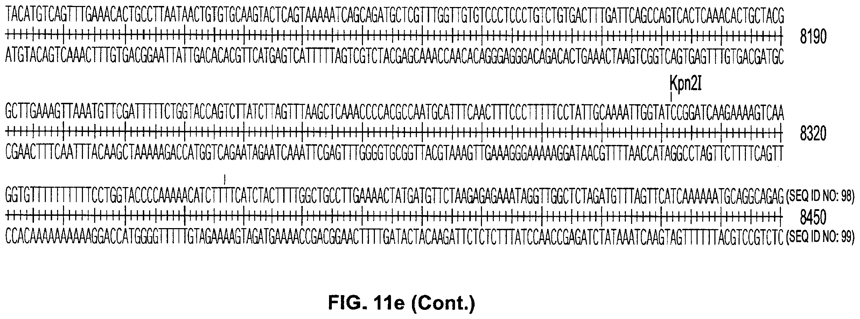

FIGs. 11A-11E depict an approach for correction of a splice site mutation in a patient with MDC1A. A patient has two mutations in the *LAMA2* gene, with one of them being a +1 mutation (c3037+1G>T) in the 5' splice site (5'ss) of exon 21 (Fig. 11A). Using Human Splicing Finder (http://www.umd.be/HSF3/) a "strength" of a non-mutated 5'ss was determined. For that, exon 21 (181 nt) +50 intronic nucleotides (231 nt total) were analyzed by HSF (Fig. 11B). Sequence position 179 presents a strength for endogenous non-mutated 5'ss (=86.17) and MaxEnt score (=6.84) (Fig. 11C). For mutated sequence (c3037+1G>T) HSF and MaxEnt scores dropped to 59.24 and -1.65, respectively (Fig. 11C). A set of new exon/intron junctions are generated by generating a double stranded break within a sequence, removing an intervening sequence containing the mutation and linking the end of the exon 21 with intronic sequences starting with 'gt' in order to constitute a possible 5'ss (Figs. 11D and 11E). In Fig. 11D, the highlighted sequence is to be removed. In Fig. 11E, a model of a new exon/intron junction after the removal of sequence highlighted in Fig. 11D.

### DETAILED DESCRIPTION OF THE DISCLOSURE

Unless defined otherwise, all technical and scientific terms used herein have the same meaning as commonly understood by one of ordinary skill in the art to which this disclosure belongs.

### Definitions

As used herein a "genome editing system" means a system that is capable of editing (e.g., modifying or altering) a target gene or DNA sequence. In an aspect, the target gene or DNA sequence is a gene or DNA sequence that contains a splice site donor mutation (i.e., a mutation in the 5' splice site (5'ss)). In an aspect, the splice site donor mutation results in the gene or DNA sequence having a splicing defect. In an aspect, the genome editing system comprises at least one nuclease that generates blunt-ended DNA breaks in a sequence-specific manner. The genome-editing system may be implemented in a cell or in an *in vitro* system. In certain aspects, the genome-editing system is used in a medicament, e.g., a medicament for correcting a splicing defect, or a medicament for treating a disease caused by a splicing defect.

As used herein, a "mutation-eliminating cut" is defined as a cut in DNA generated in a sequence-specific manner by a nuclease. The cut separates a mutation in a donor splice site of a gene from the upstream exons and introns of that gene. In one aspect, the cut is a blunt ended double strand break. A "mutation-eliminating cut site" is defined as a location in DNA at which a cut is generated in a sequence-specific manner by a nuclease to separate a mutation in a donor splice site of a gene from the upstream exons and introns of that gene. For example, a mutation-eliminating cut may occur at a Cas9 cleavage site occurring in the presence of one of the following Protospacer Adjacent Motif (PAM) sequences: NGG, NAG, NGA, NGCG, NNGRRT, NNNRRT, NNAGAA, NNNNRYAC orYG, when in the presence of a mutation-eliminating sgRNA. As used herein, a "mutation-eliminating sgRNA" is an sgRNA that in concert with a Cas9, creates a blunt ended double strand break at a mutation-eliminating cut site. The mutation-eliminating cut site may be, for example, upstream (i.e., when the DNA is in the 5' to 3' direction) of the PAM, for example, at the -3 position.

In other aspects, the mutation-eliminating cut site may be located as determined by binding by a DNA recognizing protein domain to the DNA and cleavage by a nuclease to which the DNA recognizing protein domain is fused. In another aspect, the DNA recognizing protein domain and the nuclease are joined by a linker which determines the position of a mutation-eliminating cut site. For example, with a longer linker, the mutation-eliminating cut site is positioned further away from the DNA binding domain.

As used herein, a "splice site-generating cut" is defined as a cut in an intron of a gene that is generated in a sequence-specific manner by a nuclease. The cut, in concert with a mutation-eliminating cut, results in excision of a segment of an intron, the segment containing a mutation in the donor splice site of the gene. In one aspect, the cut is a blunt ended double strand break. Upon re-ligation with the DNA ends flanking the excised segment of the intron, a functional donor splice site is constituted. This splice site-generating cut and subsequent ligation results in proper splicing of a gene such that all of the exons are expressed in RNA and translated protein. A "splice site-generating cut site" is defined as a location in the intron of a gene at which a cut is generated in a sequence-specific manner by a nuclease and, when used in concert with a mutation-eliminating cut, results in excision of a segment of an intron, the segment containing a mutation in the donor splice site of the gene. For example, a splice site-generating cut may occur at a Cas9 cleavage site occurring in the presence of one of the following Protospacer Adjacent Motif (PAM) sequences: NGG, NAG, NGA, NGCG, NNGRRT, NNNRRT, NNAGAA, NNNNRYAC or YG, when in the presence of a splice site-generating sgRNA. As used herein, a "splice site-generating sgRNA" is an sgRNA that in concert with a Cas9, creates a blunt ended double strand break at a splice site-generating cut site. The mutation-eliminating cut site may be, for example, upstream (i.e., when the DNA is in the 5' to 3' direction) of the PAM, for example, at the -3 position.

In other aspects, the splice site-generating cut may occur at a location as determined by binding by a DNA recognizing protein domain to the DNA and cleavage by a nuclease to which the DNA recognizing protein domain is fused. In another aspect, the DNA recognizing protein domain and the nuclease are joined by a linker which determines the position of a splice site-generating cut site. For example, with a longer linker, the splice site-generating cut site will be positioned further away from the DNA binding domain.

As used herein, "homology directed repair-independent," or "HDR-independent" refers to a process of modifying (e.g., repairing) DNA that does not involve homology directed repair (HDR). The homology directed repair-independent process can be, for example, a process that uses non-homologous end-joining to modify the DNA.

As used herein, "non-homologous end-joining" or "NHEJ" refers to ligation mediated modification of DNA (e.g., repair) that does not require a homologous template in the process. HNEJ includes, for example, canonical NHEJ (cNHEJ), alternative NHEJ (altNHEJ), microhomology-mediated end joining (MMEJ), and synthesis-dependent microhomology-mediated end joining (SD-MMEJ).

As used herein "repair" refers to modification of an intron such that proper splicing of the RNA (e.g., pre-mRNA) occurs (i.e., such that splicing occurs as it would in a naturally-occurring RNA, for example, an RNA transcribed from a gene that has no splicing mutations), and such that a polypeptide translated from such an RNA contains all the exons of a naturally-occurring polypeptide. In an aspect, the mutation causing a splicing defect is a mutation in the donor splice site. As used herein, the repair does not have to be a repair (e.g., correction) of the mutated splice donor site. Instead, for example, the repair may involve excision of an intron fragment containing the mutated donor splice site and ligation of the flanking ends of the DNA to create or constitute a new donor splice site in that gene, resulting in proper splicing of the gene.

As used herein, the first nucleotide of the intron is defined as the "+1" position. The second nucleotide of the intron is defined as the "+2" position. The third nucleotide of the intron is defined as the "+3" position. The fourth nucleotide of the intron is defined as the "+4" position. The fifth nucleotide of the intron is defined as the "+5" position. The sixth nucleotide of the intron is defined as the "+6" position.

As used herein, a "Cas9 molecule" or "Cas9 polypeptide" refers to a molecule or polypeptide, respectively, that can interact with a guide RNA (gRNA) molecule to localize to a site comprising a target domain (e.g., a DNA cut site) and a PAM sequence. Cas9 molecules and Cas9 polypeptides include naturally occurring Cas9 molecules and Cas9 polypeptides and engineered, altered, or modified Cas9 molecules or Cas9 polypeptides that differ, e.g., by at least one amino acid residue, from a reference sequence (e.g., a naturally-occurring Cas9 sequence).

As used herein, a "subject" means a higher eukaryote. The term includes, but is not limited to, mammals (e.g., humans, other primates, pigs, rodents (e.g., mice and rats or hamsters), rabbits, guinea pigs, cows, horses, cats, dogs, sheep, and goats). In certain aspects, the subject is a human. The human may be a child or an adult.

As used herein, "treat", "treating" and "treatment", means the treatment of a disease in a mammal, for example, a human, and includes inhibiting the disease (e.g., decreasing its rate of progression); regressing the disease; relieving or decreasing the severity of one or more symptoms of the disease; and/or curing the disease.

As used herein, "prevent," "preventing," and "prevention" means the prevention of a disease in a mammal, and includes inhibiting initiation of the disease; decreasing a predisposition toward the disease; and/or delaying the onset of at least one symptom of the disease.

As used herein, "about" or "approximately" means within an acceptable error range for a particular value as determined by a person skilled in the relevant art. Such an acceptable error range may depend, in part, on how the value is measured or assessed. "About" can mean, for example, within 3 or more than 3 standard deviations. "About" can mean within a percentage range of a given value. For example, the range can be ±1%, ±5%, ±10%, ±20%, ±30%, ±40% or ±50% of a given value. "About" can mean with an order of magnitude of a given value, for example, within 2-fold, 3-fold, 4-fold or 5-fold of a value.

### Systems and Compositions

As described herein, the inventors have provided genome editing systems, compositions and methods for constituting a functional donor splice site in a gene. These genome editing systems, compositions and methods for constituting a functional donor splice site in a gene may be used, for example, to correct a splicing defect in a gene or DNA sequence. In an aspect, the splicing defect is a pathogenic mutation. The mutation may be, for example, in the donor splice site or an intron that results in improper splicing of the RNA molecule transcribed from the gene or DNA sequence.

The disclosed genome editing systems, compositions and methods for constituting a functional donor splice site of a gene harnesses the HDR-independent repair pathway (e.g., via NHEJ) to joining of the DNA ends flanking the excised segment of the intron to constitute a functional donor splice site. In doing so, correction of pathogenic splicing defects occurs.

The disclosed genome editing systems comprise at least one nuclease that generates blunt-ended DNA breaks in a sequence-specific manner. The genome editing system is configured to form a first and a second blunt-ended double strand break in an intron of a gene containing a mutation in the splice donor site, resulting in excision of a segment of the intron containing the mutation. The system simultaneously joins the DNA ends flanking the excised segment of the intron to constitute a functional donor splice site. This results in the creation of a new donor splice site, which allows an RNA (e.g., a pre-mRNA) transcribed from the edited DNA to properly splice (e.g., to splice such that the RNA and the polypeptide translated from the RNA contains all of the exons of a naturally occurring polypeptide, for example, a polypeptide translated from a polynucleotide containing no splicing mutations). In an aspect, the genome editing system comprises at least two nucleases that generate blunt-ended DNA breaks in a sequence-specific manner.

In an aspect, the intron comprises a splice donor site mutation that alters splice site recognition, causing a splicing defect. Excising the segment of the intron containing the splice site donor mutation and simultaneously joining the DNA ends flanking the excised segment of the intron results in constitution of a functional donor splice site, thereby restoring splice site recognition in the intron.

In an aspect, the gene editing system uses a homology directed repair-independent mechanism to join the DNA ends flanking the excised segment of the intron to constitute a functional donor splice site. In a preferred aspect, the homology directed repair-independent mechanism utilizes non-homologous end joining. This mechanism can be used on all cell types where correction of a splicing defect is desired. This mechanism is particularly useful in post-mitotic (i.e., terminally differentiated) cells, where correction of a splicing defect is desired. Examples of post-mitotic cells, include but are not limited to, myocytes, cardiomyocytes, neurons, adipocytes, and skin cells.

In an aspect, the excising of the intron occurs at a mutation-eliminating cut site of the intron and at a splice site-generating cut site of the intron. It will be understood that the genome editing system may comprise a nuclease that generates a mutation-eliminating cut site and a splice generating cut site. In an aspect, the nuclease that generates the mutation-eliminating cut site is the same as the nuclease that generates the splice generating cut site. In another aspect, the nuclease that generates the mutation-eliminating cut site is different from the nuclease that generates the splice site-generating cut site.

The gene editing system comprises at least one nuclease that generates blunt-ended DNA breaks in a sequence-specific manner. There are a variety of systems that can be used to generate blunt-ended DNA breaks in a sequence-specific manner. For example, the system may be a Cas9 polypeptide or a variant of a Cas9 polypeptide that recognizes a Protospacer Adjacent Motif (PAM) (Cebrian-Serrano and Davies, "CRISPR-Cas orthologues and variants: optimizing the repertoire, specificity and delivery of genome engineering tools", Mamm Genome, DOI 10.1007/s00335-017-9697-4; and Karvelis et al., "Harnessing the natural diversity and in vitro evolution of Cas9 to expand the genome editing toolbox", Current Opinion in Microbiology, 2017, 37:88-94) . In a system using Cas9, the genome editing system further comprises a mutation-eliminating single guide RNA (sgRNA) and a splice site-generating sgRNA. In this system, the mutation-eliminating sgRNA and a splice site-generating sgRNA each comprise a CRISPR RNA (crRNA) portion as determined appropriate by the person skilled in the art. For example, each of the crRNAs may be about 17 to 27 nucleotides in length.

Methods for designing and making sgRNAs are known to those skilled in the art, and include internet based methods (see also, e.g., Haeussler M. et al., "Evaluation of off-target and on-target scoring algorithms and integration into the guide RNA selection tool CRISPOR", Genome Biol. 2016 Jul 5;17(1):148. doi: 10.1186/s13059-016-1012-2). For example, sgRNAs can be designed by finding a PAM motif and considering the preceding 17-27 nucleotides (depending on the Cas9 species to be used) as a target sequence for crRNA. Since it is known that the cleavage will occur at position PAM-3 one can predict where Cas9 is going to cut. Additional scoring for off-target effects can be done by multiple algorithms known to those skilled in the art.

The Cas9 nuclease may be from any species, including but not limited to, *S*. *pyogenes, S. aureus, S. thermophilus, C. jejuni,* and *F. novicida.* In one aspect, the Cas9 is from *S*. *aureus.* Cas9 nucleases generate blunt ended DNA breaks in a PAM-dependent manner, three nucleotides upstream (i.e., when the nucleotides are read in a 5' to 3' direction) of a PAM sequence (i.e., PAM -3). In another aspect, the mutation-eliminating cut site and/or the splice generating cut site is at a Cas9 cleavage site occurring in the presence of one of the following PAM nucleotide sequences: NGG (*S. pyogenes,* original), NAG (*S*. *pyogenes,* original), NGA (S. *pyogenes* EQR variant), NGCG (*S*. *pyogenes* VRER variant), NNGRRT (*S*. *aureus*)*,* NNNRRT (*S*. *aureus* KKH variant), NNAGAA (*S*. *thermophilus*)*,* NNNNRYAC (*C*. *jejuni*), YG *(F. novicida).*

In an aspect, the genome editing system may comprise a fusion polypeptide comprising a catalytically inactive (i.e., nuclease deficient) Cas9 (dCas9) polypeptide that recognizes a PAM and a nuclease that generates blunt-ended DNA breaks (e.g., such as the PAM sequences described above). dCas9 is commercially available (e.g., Novateinbio). In an aspect, the catalytically inactive dCas9 is fused to nuclease generating blunt ended DNA breaks. In such an aspect, the genome editing system comprises a left and right dCas9 that specify binding to DNA in a PAM-dependent manner, followed by a nuclease-dependent cleavage. To excise a segment of an intron containing a splice donor site mutation, two sets of fusion proteins can be used in the system, one that creates blunt-ended double strand breaks at a mutation-eliminating cut site and one that creates blunt-ended double strand breaks at splice site-generating cut site. The excision can them be repaired, for example, by non-homologous end joining.

Specific DNA cleavage (between any desired nucleotides) can be accomplished using other genome-editing systems. Zinc finger nucleases (ZFN) and TALENs are examples of such systems. In general, these systems work as a fusion polypeptide of DNA recognizing protein domains to a nuclease. Precise DNA targeting is accomplished by engineering the zinc finger portion or the TALE portion fused to a nuclease that generates blunt-ended DNA breaks in a sequence-specific manner, and DNA cleavage occurs through the nuclease portion of the fusion polypeptide. Gene editing systems comprising ZFNs and TALENs work as pairs (left and right), so in aspects in which ZFNs and TALENs are utilized, the genome editing system includes pairs of ZFN or TALENs and the nucleases contained on the ZFNs or TALENs dimerize for the DNA cleavage to occur. In one aspect, the ZFNs or TALE domains combine with a *Mlyl* nuclease, which upon dimerization generates blunt ended DNA breaks. To excise a segment of an intron containing a splice donor site mutation, two sets of fusion proteins can be used in the system, one that creates blunt-ended double strand breaks at a mutation-eliminating cut site and one that creates blunt-ended double strand breaks at splice site-generating cut site. The excision can them be repaired, for example, by non-homologous end joining.

In various aspects, a linker is positioned between the DNA recognizing protein domain (e.g., dCas9, TALE or zinc finger) and a nuclease that generates blunt-ended DNA breaks. The linker may be, for example, a polypeptide linker. The size of the linker may vary based on the distance between the location of the DNA site the DNA recognizing protein domain recognizes and the location at which the nuclease cuts the DNA in a sequence-specific manner.

In other aspects, the nuclease that cuts the DNA in a sequence-specific manner itself can directly target the DNA sequence of interest (e.g., a mutation-eliminating cut site or a splice site-generating cut site). In other aspects, the nuclease that cuts the DNA in a sequence-specific manner can be fused to a DNA binding protein or to an oligonucleotide that recognises a DNA sequence in proximity of an intended cut site.

The disclosed genome editing systems may be used to correct various splicing defects. In an aspect, the splicing site defect is in a splice donor site. The mutation in the splice donor site may occur at the +1 position of the splice donor site. In other various aspects, the mutation in the splice donor site occurs at the +2, +3, +4, +5, or +6 position of the splice donor site. For example, the splice site donor mutation can be a mutation in a gene provided in Table 1.

**Table 1: Donor Splice Mutations**

| **Name, mutation, position in the splice donor site** | **Gene(s)** | **Condition(s)** |
|---|---|---|
| CYP21A2, IVS7DS, G-C, +1 | CYP21A2 | 21-hydroxylase deficiency |
| SERAC1, IVS13DS, G-C, +1 | SERAC1 | 3-methylglutaconic aciduria with deafness, encephalopathy, and Leigh-like syndrome |
| HMBS, IVS6DS, G-C, +1 | HMBS | Acute intermittent porphyria |
| ABCD1, IVS6DS, G-A, +1 | ABCD1 | Adrenoleukodystrophy |
| JAG1, IVS23DS, G-C, +1 | JAG1 | Alagille syndrome 1 |
| HR, IVS12DS, G-A, +1 | HR | Alopecia universalis congenita |
| MHC2TA, IVS13DS, G-A, +1 | CIITA | Bare lymphocyte syndrome type 2, complementation group A |
| COL6A1, IVS3DS, G-A, +1 | COL6A1 | Bethlem myopathy 1 |
| FYCO1, IVS9DS, G-T, +1 | FYCO1 | Cataract, autosomal recessive congenital 2 |
| SBF2, IVS32DS, G-C, +1 | SBF2 | Charcot-Marie-Tooth disease, type 4B2 |
| TBX22, IVS4DS, G-A, +1 | TBX22 | Cleft palate with ankyloglossia |
| PRKCSH, IVS4DS, G-C, +1 | PRKCSH | Congenital cystic disease of liver |
| CPS1, IVS29DS, G-C, +1 | CPSi | Congenital hyperammonemia, type I |
| ELANE, IVS4DS, G-A, +1 | ELANE | Cyclical neutropenia |
| DIAPH1, IVS17DS, G-T, +1 | DIAPH1 | Deafness, autosomal dominant 1 |
| MYO15A, IVS4DS, G-T, +1 | MYO15A | Deafness, autosomal recessive 3 |
| TECTA, IVS9DS, G-A, +1 | TECTA | Deafness, neurosensory autosomal recessive 21 |
| DSPP, IVS3DS, G-A, +1 | DSPP | Dentinogenesis imperfecta - Shield's type II |
| KRT5, IVS1DS, G-A, +1 | KRT5 | Epidermolysis bullosa herpetiformis, Dowling-Meara |
| PTH1R, IVS8DS, G-A, +1 | PTH1R | Failure of tooth eruption, primary |
| AAAS, IVS11DS, G-A, +1 | AAAS | Glucocorticoid deficiency with achalasia |
| CETP, IVS14DS, G-A, +1 | CETP | Hyperalphalipoproteinemia |
| TRPM6, IVS16DS, G-A, +1 | TRPM6 | Hypomagnesemia 1, intestinal |
| DSG4, IVS3, G-T, +1 | DSG4 | Hypotrichosis 6 |
| GIF, IVS1DS, G-A, +1 | GIF | Intrinsic factor deficiency |
| ATP2A2, IVS6DS, G-A, +1 | ATP2A2 | Keratosis follicularis |
| GHR, IVS9DS, G-A, +1 | GHR | Laron syndrome with elevated serum GH-binding protein |
| GHR, IVS4DS, G-A, +1 | GHR | Laron-type isolated somatotropin defect |
| MYH7, IVS8DS, G-A, +1 | MYH7 | Left ventricular noncompaction 5 |
| SPRED1, IVS5DS, G-A, +1 | SPRED1 | Legius syndrome |
| ITGB2, IVSDS, G-A, +1 | ITGB2 | Leukocyte adhesion deficiency |
| LPA, IVS K4 TYPE 8 DS, G-A, +1 | LPA | Lipoprotein(a) deficiency, congenital |
| KCNH2, IVS3DS, G-C, +1 | KCNH2 | Long QT syndrome 2 |
| XK, IVS2DS, G-A, +1 | XK | McLeod neuroacanthocytosis syndrome |
| CDT1, IVS2DS, G-C, +1 | CDT1 | Meier-Gorlin syndrome 4 |
| ARSA, IVS4DS, G-A, +1 | ARSA | Metachromatic leukodystrophy, severe |
| COL1A1, IVS26DS, G-A, +1 | COL1A1 | Osteogenesis imperfecta type I |
| RSPO1, IVS5DS, G-A, +1 | RSPO1 | Palmoplantar hyperkeratosis and true hermaphroditism |
| SDHB, IVS1DS, G-T, +1 | SDHB | Paraganglioma and gastric stromal sarcoma |
| PIGA, IVS5DS, G-A, +1, SOMATIC | PIGA | Paroxysmal nocturnal hemoglobinuria 1 |
| AMHR2, IVS2DS, G-T, +1 | AMHR2 | Persistent mullerian duct syndrome, type II |
| CARD14, IVS3DS, G-A, +1 | CARD14 | Pityriasis rubra pilaris |
| PKD1, IVSDS, G-C, +1 | PKD1 | Polycystic kidney disease, adult type |
| HMBS, IVS1DS, G-T, +1 | HMBS | Porphyria, acute intermittent, nonerythroid variant |
| CNGB1, IVS32DS, G-A, +1 | CNGB1 | Retinitis pigmentosa 45 |
| PDE6G, IVS3DS, G-T, +1 | PDE6G | Retinitis pigmentosa 57 |
| CEP152, IVS4DS, G-C, +1 | CEP152 | Seckel syndrome 5 |
| ADA, IVS2DS, G-A, +1 | ADA | Severe combined immunodeficiency due to ADA deficiency |
| PTPRC, IVS13DS, G-A, +1 | PTPRC | Severe combined immunodeficiency, autosomal recessive, T cell-negative, B cell-positive, NK cell-positive |
| NEU1, IVSEDS, G-C, +1 | NEU1 | Sialidosis, type II |
| NSD1, IVS20DS, G-A, +1 | NSD1 | Sotos syndrome 1 |
| DNAJB2, IVS5DS, G-A, +1 | DNAJB2 | Spinal muscular atrophy, distal, autosomal recessive, 5 |
| FAM58A, IVS4DS, G-A, +1 | FAM58A | STAR syndrome |
| SFTPC, IVS4DS, G-T, +1 | SFTPC | Surfactant metabolism dysfunction, pulmonary, 2 |
| TBX3, IVS2DS, G-C, +1 | TBX3 | Ulnar-mammary syndrome |
| NODAL, IVS2DS, G-A, +1 | NODAL | Visceral heterotaxy 5, autosomal |
| MITF, IVS1DS, G-A, +1 | MITF | Waardenburg syndrome type 2A |
| BTK, IVS16DS, G-T, +1 | BTK | X-linked agammaglobulinemia |
| STS, IVS8DS, G-T, +1 | STS | X-linked ichthyosis with steryl-sulfatase deficiency |
| DLG3, IVS8DS, G-A, +1 | DLG3 | X-Linked mental retardation 90 |
| IL2RG, IVS3DS, G-A, +1 | IL2RG | X-linked severe combined immunodeficiency |
| ABCC2, IVS18DS, T-C, +2 | ABCC2 | Dubin-Johnson syndrome |
| C6, IVS15DS, T-C, +2 | C6 | C6 deficiency, subtotal |
| COL2A1, IVS51DS, T-C, +2 | COL2A1 | STICKLER SYNDROME, TYPE I, NONSYNDROMIC OCULAR |
| FERMT1, IVS3DS, T-C, +2 | FERMT1 | Kindler's syndrome |
| FRMD7, IVS3DS, T-G, +2 | FRMD7 | Infantile nystagmus, X-linked |
| GABRG2, IVS6DS, T-G, +2 | GABRG2 | Epilepsy, childhood absence 2\|Familial febrile seizures 8 |
| KRIT1, IVS2DS, T-C, +2 | KRIT1 | Cerebral cavernous malformations 1 |
| MARVELD2, IVS4DS, T-C, +2 | MARVELD2 | Deafness, autosomal recessive 49 |
| NDUFS6, IVS2DS, T-A, +2 | NDUFS6 | Mitochondrial complex I deficiency |
| OTOA, IVS12DS, T-C, +2 | OTOA | Deafness, autosomal recessive 22 |
| PLP1, IVS3DS, T-C, +2 | PLP1 | Pelizaeus-Merzbacher disease |
| PRDM5, IVS1DS, T-C, +2 | PRDM5 | Brittle cornea syndrome 2 |
| RPL5, IVS2DS, T-G, +2 | RPL5 | Aase syndrome |
| SMARCAD1, IVS1DS, T-C, +2 | SMARCAD1 | Adermatoglyphia |
| TRPM1, IVS16DS, T-C, +2 | TRPM1 | Congenital stationary night blindness, type 1C |
| TULP1, IVS7DS, T-C, +2 | TULP1 | Retinitis pigmentosa 14 |
| WAS, IVS6DS, T-G, +2 | WAS | Wiskott-Aldrich syndrome |
| SMARCAD1, IVS1DS, A-T, +3 | SMARCAD1 | Basan syndrome |
| COL1A2, IVS26DS, A-G, +3 | COL1A2 | Osteogenesis imperfecta with normal sclerae, dominant form |
| ATP7A, IVSXDS, A-T, +3 | ATP7A | Menkes disease, mild |
| F8, IVS6DS, A-G, +3 | F8 | Hereditary factor VIII deficiency disease |
| RPGR, IVS10DS, A-G, +3 | RPGR | Retinitis pigmentosa 15 |
| COLQ, IVS16DS, A-G, +3 | COLQ | Endplate acetylcholinesterase deficiency |
| BLNK, IVS1DS, A-T, +3 | BLNK | Agammaglobulinemia 4, autosomal recessive |
| NDUFV1, IVS8DS, A-C, +4 | NDUFV1 | Mitochondrial complex I deficiency |
| ENG, IVS3DS, A-G, +4 | ENG | Osler hemorrhagic telangiectasia syndrome |
| HSPG2, IVS64DS, A-G, +4 | HSPG2 | Schwartz Jampel syndrome type 1 |
| SMARCAD1, IVS1DS, G-C, +5 | SMARCAD1 | Adermatoglyphia |
| OCLN, IVS5DS, G-A, +5 | OCLN | Band-like calcification with simplified gyration and polymicrogyria |
| RFX5, IVS2DS, G-A, +5 | RFX5 | Bare lymphocyte syndrome, type II, complementation group c |
| COL1A2, IVS11DS, G-A, +5 | COL1A2 | Ehlers-Danlos syndrome, autosomal recessive, cardiac vascular form |
| FANCB, IVS7DS, G-A, +5 | FANCB | Fanconi anemia, complementation group B |
| F8, IVS12DS, G-A, +5 | F8 | Hereditary factor VIII deficiency disease |
| IKBKG, IVS6DS, G-A, +5 | IKBKG | Hypohidrotic ectodermal dysplasia with immune deficiency |
| TRPM6, IVS8DS, G-C, +5 | TRPM6 | Hypomagnesemia 1, intestinal |
| HPRT, IVS7DS, G-A, +5 | HPRT1 | Lesch-Nyhan syndrome |
| COL9A3, IVS3DS, G-A, +5 | COL9A3 | Multiple epiphyseal dysplasia 3 |
| COL1A1, IVS14DS, G-A, +5 | COL1A1 | Osteogenesis imperfecta, recessive perinatal lethal |
| PROS1, IVS10DS, G-A, +5 | PROS1 | Protein S deficiency |
| NPHP3, IVS13DS, G-A, +5 | NPHP3 | Renal-hepatic-pancreatic dysplasia |
| ADAMTS13, IVS13DS, G-A, +5 | ADAMTS13 | Upshaw-Schulman syndrome |
| DLG3, IVS6DS, G-A, +5 | DLG3 | X-Linked mental retardation 90 |
| ATP7A, IVS6DS, T-A, +6 | ATP7A | Cutis laxa, X-linked |
| SMN1, IVS7DS, T-G, +6 | SMN1 | Kugelberg-Welander disease, spinal Muscular Atrophy Type III |

In the disclosed genome editing system, the splice site-generating cut site is positioned before a branch splice point for an adjacent exon. Methods for determining branch splice point for an adjacent exon are known to those skilled in the art (see, e.g., Mercer, Genome Research, 2015 PMID: 25561518; Reed, PNAS, 1988 PMID: 3060403). For example, the splice site-generating cut site may be positioned at least, but not limited to, 25, 50, 100, 150, 200, 300, 400, 500, 600, 700, 800, 900 or 1000 base pairs from the mutation-eliminating cut site, provided the splice site-generating cut site is positioned before the branch splice point for an adjacent exon.

In one aspect, the genome editing system comprises a mutation-eliminating single guide RNA (sgRNA); a splice site-generating sgRNA and a Cas9 molecule that recognizes a PAM having the nucleotide sequence NNGRRT, wherein the mutation-eliminating cut site of the intron is positioned 3 nucleotides in the 5' direction of the end of the PAM.

In one aspect, mutation-eliminating cut sites and splice site-generating cut sites are determined by predicting splice sites in a nucleotide sequence. Methods for predicting splices sites are generally known in the art and include the use of tools such as Human Splicing Finder (HSF) (http://www.umd.be/HSF3/), the MaxEntScan donor model and/or the DeepScan donor model. Based on the sequence of the nucleotides at in in proximity to the mutation-eliminating cut sites and splice site-generating cut sites the appropriate nuclease can be selected and the cuts sites can be generated. Excision of the identified intron segment (and the mutation) and simultaneous joining together of the DNA ends flanking the excised intron segment result in constitution of a functional donor splice site, such that the gene can now be properly spliced and a functional protein can be generated upon translation.

In various aspects, the genome editing system further comprises a delivery vehicle for delivery of the other components of the system (e.g., for delivery of the at least one nuclease that generates blunt-ended DNA breaks in a sequence-specific manner, and one or more of the sgRNAs, if used in the system). In an aspect, polynucleotides coding for the at least one nuclease that generates blunt-ended DNA breaks in a sequence-specific manner are in a vector. In another aspect, when the nuclease is Cas9, the vector further comprises a mutation-eliminating single sgRNA or a splice site-generating sgRNA. The delivery vehicle may be an adeno-associated virus (AAV) vector, for example, an AAV2, AAV3, AAV4, AAV5, AAV6, AAV7, AAV8 or AAV9 vector. In a preferred aspect, the delivery vehicle is an AAV9 vector.

In certain aspects, a mutation-eliminating sgRNA is present on a first nucleic acid molecule, e.g. a first vector (e.g., a first viral vector, such as a first AAV vector); and a splice generating sgRNA is present on a second nucleic acid molecule e.g., a second vector (e.g., a second vector, for example, a second AAV vector).

In certain aspects, a mutation-eliminating sgRNA and polynucleotides encoding a nuclease that generates blunt-ended DNA breaks in a sequence-specific manner are present on one nucleic acid molecule, e.g., one vector (e.g., one viral vector, such as one AAV vector). In certain aspects, a mutation-eliminating sgRNA and polynucleotides encoding a nuclease that generates blunt-ended DNA breaks in a sequence-specific manner are present on different vectors. For example, a mutation-eliminating sgRNA is present on a first nucleic acid molecule, e.g. a first vector, (e.g., a first viral vector, for example, a first AAV vector); and polynucleotides encoding a nuclease that generates blunt-ended DNA breaks in a sequence-specific manner are present on a second nucleic acid molecule, e.g., a second vector (e.g., a second vector, such as a second AAV vector).

In certain aspects, a splice generating sgRNA and polynucleotides encoding a nuclease that generates blunt-ended DNA breaks in a sequence-specific manner are present on one nucleic acid molecule, e.g., one vector, (e.g., one viral vector, for example, one AAV vector). In certain aspects, a splice generating sgRNA and polynucleotides encoding a nuclease that generates blunt-ended DNA breaks in a sequence-specific manner are present on different vectors. For example, a splice generating sgRNA is present on a first nucleic acid molecule, e.g. a first vector (e.g., a first viral vector, such as, a first AAV vector); and polynucleotides encoding a nuclease that generates blunt-ended DNA breaks in a sequence-specific manner are present on a second nucleic acid molecule, e.g., a second vector (e.g., a second vector, for example, a second AAV vector).

The vectors may comprise a promoter operably linked to a mutation-eliminating sgRNA, a splice generating sgRNA or polynucleotides encoding a nuclease that generates blunt-ended DNA breaks in a sequence-specific manner. Where a vector contains more than one nucleotide sequence (e.g., a mutation-eliminating sgRNA and polynucleotides encoding a nuclease that generates blunt-ended DNA breaks in a sequence-specific manner or a splice generating sgRNA and polynucleotides encoding a nuclease that generates blunt-ended DNA breaks in a sequence-specific manner) the vector may further comprise a second promoter operably linked to the second nucleotide sequence. When two promoters are employed in a vector, the promoter may be the same or they may be different. Promoters for use in expression vectors are known to those skilled in the art.

In addition to AAVs, the vector may comprise plasmid DNA, encoding CRISPR/Cas9 components (Cas9 + sgRNA) as a delivery vehicle. Alternatively, the delivery vehicle can comprise RNA, where polynucleotides encoding Cas9 and sgRNAs are comprised of RNA molecules. Alternatively, the delivery vehicle can be in an RNP (ribonucleoprotein) form, where Cas9 is delivered in a complex with sgRNA (RNA form).

In other aspects, the delivery vehicle is a non-viral delivery vehicle, for example, a liposome, such as a cationic liposome, or polymeric nanoparticles. Methods for delivering the disclosed gene editing system to a cell are known in the art.

In an aspect, the genome editing system comprises a *S*. *aureus* Cas9 molecule that recognizes a PAM, a mutation-eliminating sgRNA and a splice site-generating sgRNA. In an aspect, the mutation-eliminating sgRNA comprises the nucleotide sequence aaaacatatatataatacatatGGT (SEQ ID NO: 1). The genome editing system targets a pathogenic mutation in the *Lama2* gene, wherein the mutation is a c.417+1 g→a mutation. In certain aspects, the mutation is in a subject having merosin deficient congenital muscular dystrophy (MDC1A).

Compositions comprising one or more components of the gene editing system are also disclosed. The composition may comprise, for example, at least one nuclease that generates blunt-ended DNA breaks in a sequence-specific manner. The composition is configured to form a first and a second blunt-ended double strand break in an intron of a gene, wherein the intron comprises a splice donor site mutation that alters splice site recognition, thereby excising a segment of the intron and simultaneously joining DNA ends flanking the excised segment of the intron to constitute a functional donor splice site.

The nuclease may be in a fusion polypeptide comprising a DNA recognizing protein domain and the nuclease. For example, the fusion polypeptide may comprise a dCas9 molecule that is fused to a nuclease that generates blunt-ended DNA breaks in a sequence-specific manner as described herein. In an aspect, zinc finger polypeptides are the DNA recognizing protein domain. Similarly, the fusion polypeptide may comprise a TALE polypeptide as the DNA recognizing protein domain. When the nuclease portions of the fusion polypeptides dimerize, the pair of fusion polypeptides generates blunt-ended DNA breaks.

The composition may further comprise a linker between the catalytically inactive Cas9 and the nuclease or between a DNA recognizing protein domain and the nuclease, as described herein.

Where the nuclease is a Cas9 molecule, the composition further comprises a mutation-eliminating sgRNA and a splice site-generating sgRNA. Such compositions may further comprise a delivery vehicle such as a vector (including a viral vector) as described herein or liposomes or nanoparticles. The compositions may be used in the genome editing systems disclosed herein. Each of the mutation-eliminating sgRNA and the splice site-generating sgRNA may comprise a CRISPR RNA (crRNA) portion that is 17 to 27 nucleotides in length.

### Methods of Constituting a Functional Donor Splice Site in a Gene

Methods of constituting a functional donor splice site in a gene, where the gene comprises a mutation in a donor splice site that alters splice site recognition are also provided. The method comprises delivering a genome editing system to the gene. The genome editing system comprises at least one nuclease that generates blunt-ended DNA breaks in a sequence-specific manner. The genome editing system is configured to form a first and a second blunt-ended double strand break in an intron of a gene, wherein the intron comprises a splice donor site mutation that alters splice site recognition. This results in excision of a segment of the intron and simultaneously joining of DNA ends flanking the excised segment of the intron to constitute a functional donor splice site. This results in restored splice site recognition in the intron. The joining of the DNA ends flanking the excised segment of the intron to constitute a functional donor splice site occurs through a homology directed repair-independent mechanism, for example, through non-homologous end joining.

Other features of the genome editing system for using in a method of constituting a functional donor splice site in a gene are as described herein.

In an aspect, the gene with the donor splice mutations is a gene listed in Table 1.

Methods of detecting constitution of a functional donor splice site in a gene, where the gene comprises a mutation in a donor splice site that alters splice site recognition are known to those skilled in the art. For example, RT-PCR can be performed on the cDNA flanking the exon affected by the splicing defect and gel electrophoresis can be used to determine if a functional donor splice site has been constituted. Western blot analysis may be performed to determine of a full-length protein has been generated, indicating successful constitution of a function donor splice site. Methods for performing these techniques are known in the art.

### Cells

Isolated cells comprising a gene whose mutation at a splice donor site has been repaired according to the method for constituting a functional donor splice site in a gene, where the gene comprises a mutation in a donor splice site that alters splice site recognition are also provided.

The cell may be a post-mitotic cell, for example, a myocyte, cardiomyocyte, neuron, adipocyte, or skin cell.

Components of a genome editing system as described herein are can be delivered by a vector, for example, an AAV vector as described herein. Alternatively, the components can be delivered using non-viral systems, for example, liposomes or nanoparticles. Delivery into cells can occur using techniques known in the art, including, but not limited to microinjection, electroporation, lipid-mediated transfection, RNP-mediated delivery, RNA-mediated delivery, or a combination thereof.

Methods of treating a disease or condition caused by a mutation in a splice donor site in a gene of a subject

Methods for treating a disease or condition caused by a mutation in a splice donor site in a gene of a subject in a subject in need of such treatment are also provided. The method comprises delivering a genome editing system to the intron. The genome editing system comprises at least one nuclease that generates blunt-ended DNA breaks in a sequence-specific manner. The genome editing system is configured to form a first and a second blunt-ended double strand break in an intron of a gene, wherein the intron comprises a splice donor site mutation that alters splice site recognition. This results in excision of a segment of the intron and simultaneously joining of DNA ends flanking the excised segment of the intron to constitute a functional donor splice site. This results in restored splice site recognition in the intron. The joining of the DNA ends flanking the excised segment of the intron to constitute a functional donor splice site occurs through a homology directed repair-independent mechanism, for example, through non-homologous end joining.

Other features of the genome editing system for using in a method of treating a disease or condition caused by a mutation in a splice donor site in an intron in a subject are as described herein.

In an aspect, the subject has a disease or condition caused by a donor splice mutation in a gene as listed in Table 1.

In certain aspects, the treatment is initiated prior to onset of the disease. In certain aspects, the treatment is initiated after onset of the disease. In certain aspects, the treatment is initiated prior to onset of a symptom of the disease. Alternatively, the treatment is initiated at onset of one or more symptoms of the disease. In certain aspects, the treatment is initiated after onset one or more symptoms of the disease.

Components of a genome editing system as described herein are can be delivered by a vector, for example, an AAV vector as described herein. Alternatively, the components can be delivered using non-viral systems, for example, liposomes or nanoparticles. Delivery into cells can occur using techniques known in the art, including, but not limited to microinjection, electroporation, lipid-mediated transfection, peptide-mediated delivery, or a combination thereof. The dose(s) of components of the system to use for treating a disease or condition caused by a mutation in a splice donor site of a gene will depend on the mode of delivery and is within the skill of the skilled person to determine.

Components of the genome editing system can be delivered systemically. Systemic modes of administration include oral and parenteral routes, including but not limited to intravenous, intra-arterial, intramuscular, intradermal, subcutaneous, intranasal, and intraperitoneal routes.

Components of the genome editing system can be delivered locally. Local modes of administration include injection directly into one or more specific tissues. In one aspect, local delivery occurs by direct injection in a muscle on the subject.

### Kits

The present disclosure contemplates kits for carrying out the methods disclosed herein. Such kits typically comprise two or more components required for constituting a functional donor splice site in a gene, where the gene comprises a mutation in a donor splice site that alters splice site recognition. Components of the kit include, but are not limited to, one or more of compounds, reagents, containers, equipment and instructions for using the kit. Accordingly, the methods described herein may be performed by utilizing pre-packaged kits provided herein. In one aspect, the kit comprises at least one nuclease that generates blunt-ended DNA breaks in a sequence-specific manner. In some aspects, the instructions comprise one or more protocols for constituting a functional donor splice site in a gene, where the gene comprises a mutation in a donor splice site that alters splice site recognition. In some aspects, the kit comprises one or more controls.

The following examples illustrative of the disclosure are provided.

### Example 1: Materials and Methods

### Cloning and Virus Production

Each sgRNA shown in Table 2, was cloned into pX601 plasmid (Addgene 61591) containing codon optimized *S*. *aureus* Cas9 (3).

**Table 2: sgRNA Sequences Used in the Study**

| **Name** | | **Sequences (5' --> 3')** | **PAM** | **ID number** |
|---|---|---|---|---|
| sgRNA 1 | Top | aacatatataatacatatGGT (SEQ ID NO: 2) | ATGGAT | RDC 495 |
| | Bottom | ACCatatgtattatatatgtt (SEQ ID NO: 3) | | RDC 496 |
| sgRNA 2 | Top | taggaagtagaaacttaggtc (SEQ ID NO: 4) | aagaat | RDC 501 |
| | Bottom | gacctaagtttctacttccta (SEQ ID NO: 5) | | RDC 502 |
| sgRNA 3 | Top | agagggcagagtcttgacatgtg (SEQ ID NO: 6) | tggagt | RDC 503 |
| | Bottom | cacatgtcaagactctgccctct (SEQ ID NO: 7) | | RDC 504 |
| sgRNA 4 | Top | ccctggccacagaatgctgtc (SEQ ID NO: 8) | tggga | RDC 1458 |
| | Bottom | gacagcattctgtggccaggg (SEQ ID NO: 9) | | RDC 1459 |
| sgRNA 5 | Top | atgaaagatgtataaggagtt (SEQ ID NO: 10) | aagaat | RDC 1460 |
| | Bottom | aactccttatacatctttcat (SEQ ID NO: 11) | | RDC 1461 |
| sgRNA 6 | Top | agtcttgacatgtgtggagtg (SEQ ID NO: 12) | aagaa | RDC 1462 |
| | Bottom | cactccacacatgtcaagact (SEQ ID NO: 13) | | RDC 1463 |
| sgRNA 7 | Top | gtgaagaaactggaaacagtt (SEQ ID NO: 14) | cagaa | RDC 1464 |
| | Bottom | aactgtttccagtttcttcac (SEQ ID NO: 15) | | RDC 1465 |
| sgRNA 8 | Top | ggctgacttagagatatggtt (SEQ ID NO: 16) | tagaa | RDC 1466 |
| | Bottom | aaccatatctctaagtcagcc (SEQ ID NO: 17) | | RDC 1467 |
| sgRNA 9 | Top | taatagagtaaatactgtgtt (SEQ ID NO: 18) | tggggt | RDC 1468 |
| | Bottom | aacacagtatttactctatta (SEQ ID NO: 19) | | RDC 1469 |
| sgRNA 10 | Top | ggaagatgcttatgttttgtt (SEQ ID NO: 20) | gaggg | RDC 1470 |
| | Bottom | aacaaaacataagcatcttcc (SEQ ID NO: 21) | | RDC 1471 |
| sgRNA 11 | Top | cacactgaaaagtaacaccaa (SEQ ID NO: 22) | gtgaat | RDC 1472 |
| | Bottom | ttggtgttacttttcagtgtg (SEQ ID NO: 23) | | RDC 1473 |

As control, a GFP plasmid was constructed by replacing the SaCas9 cassette in pX601 with eGFP codon from pEGFP-C1 plasmid (Clontech Takara) using Age1 and EcoR1 directional cloning. 2100 bp stuffer sequence was incorporated into Xba1 site of the vector to maintain AAV genome packaging capacity. The pX601-GFP, -sgRNA1 and -sgRNA2 plasmids were produced as Adeno-associated virus serotype 9 (AAV9), concentrated and quantified by quantitative PCR (Vigene Biosciences). Injection-ready virus solution containing 7.5×10¹¹ viral genome/vector was prepared and brought to 30 µl with 1X PBS (Gibco).

### Cell Culture and Guide Screening

Primary myoblasts were isolated from EDL muscle of C57BL/6J wild-type and *dy^{2J}*/*dy^{2J}* mice (18) and maintained in DMEM supplemented with 1% Chicken Embryo Extract (Gemini Biosciences), 10% Horse Serum, 1% Penicillin/Streptomycin, 1% L-glutamine (All from Gibco unless indicated otherwise) at 37°C with 5% CO₂. Transfection was performed using Amaxa Basic Nucleofector Kit VPI-1002 (Lonza) in Nucleofector device (Lonza) under program number U-023. Cells were grown for 72 hours and genomic DNA was isolated with Blood and Tissue DNA Isolation Kit based on manufacturer's protocol (Qiagen). The activity of guides combination to remove the intervening region was assessed by PCR using primers in *Lama2* exon 2 (RDC 551) and intron 2 (RDC 552) flanking the genomic deletion. The sequences used are shown in Table 3.

**Table 3: Frequency of Different Genomic DNA Products after Cas9-mediated Deletion**

| Sequences | Percentage |
|---|---|
| **AATCCATACC***GTCAAGAATG* (SEQ ID NO: 24) | 56.29% |
| AATCCATAC - - TCAAGAATG (SEQ ID NO: 25) | 13.92% |
| AATCCATACC - TCAAGAATG (SEQ ID NO: 26) | 11.41% |
| AATCCATACg - - CAAGAATG (SEQ ID NO: 27) | 0.29% |
| AATCCATACtGTCAAGAATG (SEQ ID NO: 28) | 0.23% |

### Animal Studies

The *dy^{2J}*/*dy^{2J}* and C57BL/6J mice were purchased from the Jackson Laboratory and maintained in the Toronto Centre for Phenogenomics (TCP). All animal experiments were performed according to Animal Use Protocol number 16-0234H. No statistical methods were used to predetermine sample size. Intramuscular injection was performed on 3-weeks old *dy^{2J}*/*dy^{2J}* mice under isofluorane anesthesia. Treatments were randomized among littermates. No blinding was done in the intramuscular treatment. For systemic administration experiments involving neonatal pups, breeding cages were monitored daily for birth and the entire litter of P2 newborns were injected with either GFP- or guide-containing vectors. Mice were genotyped (19) and weaned at the age of 3 weeks old, where randomization occurred and all investigators were subsequently blinded for the different treatment groups throughout the study until the time of necropsy.

### Functional Tests

Open field activity test and assessment of *in vivo* muscle force were performed on 10 weeks-old mice (for both intraperitoneal and temporal vein cohorts) at the Lunenfeld-Tanenbaum Research Institute's Centre for Modeling Human Disease Mouse Phenotyping Facility. For the open field test, mice were placed in the frontal center of a transparent Plexiglas open field (41.25 cm × 41.25 cm × 31.25 cm) illuminated by 200 Ix. Trained operator, who is unaware of the nature of the projects and treatments, performed the experiments. The VersaMax Animal Activity Monitoring System recorded vertical activities and total distance travelled for 20 minutes per animal.

*In vivo* muscle contraction test was performed using 1300A: 3-in-1 Whole Animal System and analyzed using Dynamic muscle control/analysis (DMC/DMA) High throughput software suite (Aurora Scientific). The mice were anaesthetised with intraperitoneal injection of ketamine/xylazine cocktail at 100 mg/kg and 10 mg/kg of body weight, respectively. Contractile output was measured via percutaneous electrodes that stimulate specific nerves innervating the plantar flexors. Specific tetanic force (200 ms of 0.5-ms pulses at 125 Hz) was recorded and corrected to body weight.

### RNA Isolation, RT-PCR, Quantitative PCR (qPCR)

RNA from cultured cells were isolated using Trizol (Invitrogen) according to manufacturer's instruction. For mouse tissues, muscles were sectioned at 10 µm thin, collected in 1.4 mm Zirconium Beads pre-filled tubes (OPS Diagnostics) and homogenized using a MagNA Lyser (Roche Diagnostics) using Trizol (Invitrogen). N6-primed cDNA was synthesized from 1000 ng of RNA using SuperScript III (Invitrogen). PCR amplification to detect exon inclusion was performed using Multiplex PCR mix (Qiagen) and primers in exons 1 and 4 (RDC 449 and 450), with sequences shown in Table 2.

qPCR was performed using Fast SYBR Green Master Mix on Step One Plus Real Time PCR (Applied Biosystems). Control PUC-Lama2 plasmid was generated by directional cloning in our laboratory. Briefly, exon 1-7 *Lama2* cDNA was amplified from wild type muscles using primers RDC 1411 and RDC 1479, and cloned into PUC19 plasmid at BamH1 restriction sites. The expression of products with exon 2 inclusion was calculated using primers in exon 2/exon 3 junctions, and normalized to expression of products amplified with exon 4. Copy number of all samples was calculated relative to PUC-Lama2 plasmid control.

### Protein Isolation and Western Blot

Sectioned muscles collected in the Zirconium beads tubes were homogenized in 500 µl of RIPA homogenizing buffer (50 mM Tris HCI pH 7.4, 150 nM NaCl, 1 mM EDTA supplemented with protease inhibitor cocktails (Roche)) and lysed with a MagNA Lyser. Subsequently, 500 µl of RIPA double detergents buffer (2% deoxycholate, 2% NP40, 2% Triton X-100 in RIPA homogenizing buffer) was added to the lysates, which were then incubated for 45 minutes at 4°C with gentle agitation and centrifuged for 10 minutes at 13,000 rpm. The protein concentration was measured using BCA Assay (Thermo Scientific). Protein was separated on 3-8% Tris-Acetate gel and transferred using the Novex system (Invitrogen). The primary antibodies used were rat monoclonal anti-Lama2 (Abcam 11576 4H8-2, 1:500), rabbit polyclonal anti-tubulin (Abcam 4074, 1:5000), and mouse monoclonal anti-HA (Abcam 18181, 1:2500).

### Immunofluorescence, H&E and Picrosirius Red Staining

Antibodies used for immunofluorescence stainings were rat monoclonal anti-Lama2 (Abeam 11576 4H8-2, 1:500), mouse monoclonal anti-Neurofilament H (NF-H, Biologend 801601 SMI 31, 1:1000), FITC-conjugated rat anti-mouse Cd11b clone M1/70 (BD Pharmingen 553310, 1:50) and imaged using either Zeiss Axiovert 200M or Nikon TE-2000 epifluorescence microscopes, or Olympus 1X81 quorum spinning disk confocal microscope. Perkin Elmer Velocity software was used for image acquisition.

Hematoxylin and eosin (H&E) staining was performed using standard protocol and Picrosirius Red staining was performed at Pathology Laboratory, the Hospital for Sick Children, Toronto. 300 fibers obtained from 4 different field of views per animal were analyzed using imaged 1.51h software for minimum Feret's diameter and cross-sectional area. Fibrosis quantification was performed from Sirius Red-stained (for intraperitoneal cohort) and H&E-stained (for temporal vein cohort) images by image-segmentation using imaged 1.51h software.

### Off-target Analysis by T7 Endonuclease Assay

Top 10 predicted off-target sites were computed using Benchling CRISPR Design tool (20). Genomic DNA isolated from treated- and control mice were PCR-amplified using primers targeting loci corresponding to each hit, as listed in Table 2.

Purified PCR products were then digested using T7 Endonuclease I (NEB) based on the manufacturer's instruction.

### Deep Sequencing

Genomic DNA extracted from tibialis anterior (TA) muscles from the temporal vein cohorts (Qiagen Blood and Tissue kit) were amplified using oligos RDC 1474 and RDC 1461 as shown in Tables 2 and 4. A second round of PCR was performed to add 5' and 3' end barcodes, and sequenced with 125 bp paired-end reads on Illumina HiSeq 2500 at the Toronto Center for Applied Genomics (TCAG). Bioinformatic analyses were done similarly as previously described (21).

**Table 4: Primers Used for PCR, RT-PCR, and qPCR**

| **Name** | **Sequences** | **ID number** | **Used for** | **Reference to figure(s)** |
|---|---|---|---|---|
| Lama2-guide screen F | AAG GAC CCG AGA TGT ACT GC (SEQ ID NO: 29) | RDC 551 | Guide/deletion screening | Fig. 1d |
| Lama2-guide screen R | TGG CCT GTT CTT AGT GCT TG (SEQ ID NO: 30) | RDC 552 | | |
| Lama2-guide screen 1-F | GTGCCGAATCTGCAA TCAGAACAGC (SEQ ID NO: 31) | RDC 1474 | Guide/deletion screening ddPCR | Fig. S1 |
| Lama2-guide screen 1-R | cactccacacatgtcaagactct gc (SEQ ID NO: 32) | RDC 1475 | | |
| Lama2-guide screen2-F | GAAAAAGGACCCGAG ATGTACTGCAAG (SEQ ID NO: 33) | RDC 1476 | Guide/deletion screening | Fig. S1 |
| Lama2-guide screen2-R | ctatatcaaaattgattttgaaat gcaatgaagctg (SEQ ID NO: 34) | RDC 1477 | | |
| Lama2-ex1 Fw 2 | CAG TCC CAA GCG CAT CAA C (SEQ ID NO: 35) | RDC449 | Exon inclusion detection | Fig. 1d |
| Lama2-ex5 Fw | GAATGTAGCGAGCAG AGGTGAATTC (SEQ ID NO: 36) | RDC 1478 | | |
| Lama2-ex4 Rev | TGG CTG CCT TCA CAA TTA CG (SEQ ID NO: 37) | RDC 450 | Exon inclusion detection | Figs. 2b, 3a, 3b, 3i,4b, S1, S6, S7 |
| Lama2-5'UTR-F | aggagtggatctgctccgg (SEQ ID NO: 38) | RDC 1411 | Generation of PUC-Lama2 | Figs. 3c, 4c |
| Lama2-ex7 Rev1 | CACACTCAGACTTGG TGAGGAAG (SEQ ID NO: 39) | RDC 1479 | | |
| Lama2-ex1ex2-gqPCR-F | GCATCAACAGAGAGG TTTATTTCCTG (SEQ ID NO: 40) | RDC 1480 | qPCR | Figs. 3c, 4c |
| Lama2-ex3-qPCR-R | CAATAGCATTCGTAAT CGGGTGC (SEQ ID NO: 41) | RDC 1481 | | |
| Lama2-ex4ctrl-qPCR-F | GTAATTGTGAAGGCA GCCAATTC (SEQ ID NO: 42) | RDC 1482 | | |
| Lama2-ex4ctrl-qPCR-R | TGCGGGGATAGATAT TGTAGAGG (SEQ ID NO: 43) | RDC 1483 | | |

### Quantitative Droplet Digital PCR (ddPCR)

ddPCR was performed on temporal vein samples using a QX200^{™} Droplet Digital^{™} PCR System with QX200^{™} ddPCR EvaGreen Supermix (BioRad). Three-steps ddPCR was performed with the following conditions: 95°C (10 mins), 94°C (30 sec), 62°C (30 sec), 72°C (1 min) for 45 cycles, and 98°C (10 mins) using Lama2 guide screen 1F (RDC 1474) and 1R (RDC 1475). The data were analyzed using QuantaSoft Pro (BioRad).

### Example 2: Exon Inclusion Strategy Enables Restoration of the Full-length Lama2 Expression

Mammalian donor splice sites for major U2-type introns have a 9 nucleotides long consensus sequence A/C-A-G-g-t-a/g-a-g-u and are mainly recognized by canonical base pairing to the 5' end of U1 small nuclear RNA (25). The first two intronic nucleotides (gt) are essentially invariant, whereas certain flexibility is allowed at all other positions. Targeted CRISPR/Cas9-mediated removal of an intronic region containing a pathogenic mutation was used to enable joining of *Lama2* exon 2 with a 'gt' dinucleotide, leading to reconstitution of a functional donor splice site.

A mouse model of MDC1A, *dy^{2J}*/*dy^{2J}* harboring a splice site mutation in intron 2 of *Lama2,* annotated as c.417+1 g→a, which results in skipping of exon 2 and truncation of the N-terminal domain of the protein (Fig. 1A) (32, 33) was used to determine if a functional donor splice site could be obtained. SaCas9 PAM sequences (NNGRR(T)) in proximity to the g→a mutation in *Lama2* were searched for and one located at the end of the exon 2 was found (Fig. 1B). The SaCas9 was predicted to generate a DSB three nucleotides upstream of the PAM sequence 3, which in this case was immediately upstream of the mutation (Fig. 1B). sgRNA targeting this region was designed and annotated as sgRNA1, which serves as a mutation-eliminating sgRNA.

Approximately 1400 nucleotides in a proximal region of intron 2 were evaluated for SaCas9 targets that would generate genomic deletions in combination with sgRNA1 and, following NHEJ, create potential donor splice sites (splice site-generating cut) (Fig. 1C). Ten out of thirteen possible splice site-generating sgRNAs (sgRNA2-sgRNA11) were tested in *dy^{2J}*/*dy^{2J}* myoblasts by co-transfection with sgRNA1 (Figs. 1D, 1G ).

RT-PCR analysis revealed the presence of an amplicon similar to the wild-type control for sgRNA2, sgRNA3 and sgRNA5, indicating an inclusion of the exon 2 of *Lama2* after generation of intronic deletions (Figs. 1D, 1H). Among these three functional 'splice generating sgRNAs', the sgRNA2 target was confirmed to be closest to the exon-intron boundary and Sanger sequencing confirmed that full-length exon 2 was successfully incorporated in the *Lama2* mRNA in sgRNA1+sgRNA2-treated *dy^{2J}*/*dy^{2J}* myoblasts (Fig. 1F).

### Example 3: Restoration of Full-length Lama2 and Improvement of Muscle Pathology Following Intramuscular Administration of AAV9-CRISPR

To assess the impact of the full-length Lama2 restoration in skeletal muscle, Cas9 and sgRNAs were delivered into the right tibialis anterior (TA) muscles of 3-week old *dy^{2J}*/*dy^{2J}* mice using adeno-associated virus serotype 9 (AAV9) (Fig. 2A). Mice injected with AAV9 carrying a GFP expression cassette served as controls. Three weeks post-injection, expression of the HA-tagged Cas9 or GFP was only detected in the injected right TA (Fig. 2E).

RT-PCR analysis successfully identified transcripts with inclusion of exon 2 in all right TA muscles treated with AAV9-Cas9-sgRNA1 and -sgRNA2, but not in contralateral muscles and AAV9-GFP-treated mice (Fig. 2B).

The expression of Lama2 was analyzed by western blot, and it was observed that in all *dy^{2J}*/*dy^{2J}*-isolated muscles, a truncated form of Lama2 was present, whereas a slightly larger full-length protein was expressed in the TA muscles injected with AAV9-Cas9-sgRNA1 and -sgRNA2 combination (Fig. 2C), demonstrating that transcripts with exon 2 inclusion were successfully translated into full-length Lama2 protein.

To assess the localization of the restored Lama2, immunofluorescence staining was performed on the treated TA muscles. Lama2 immunoreactivity was detected at the sarcolemma of muscles fibers, similar to the wild-type controls (Fig. 2D). Importantly, the histological analyses of treated skeletal muscles exhibited considerably improved muscle architecture compared to the contralateral control TA as shown by reduced areas of fibrotic and infiltrating inflammatory cells (Fig. 2D).

This demonstrated the successful inclusion of the previously missing exon 2 and restoration of the full-length Lama2 protein at the sarcolemma of the muscle, which led to overall improvement of muscle histopathology supporting the therapeutic potential of this treatment strategy in the *dy^{2J}*/*dy^{2J}* mice.

### Example 4: Restoration of Lama2 in Neonatal Mice Through Intraperitoneal Administration of AAV9-Cas9-sgRNA1 and -sgRNA2

It was next asked whether the dystrophic pathology, which is already apparent at pre-weaning age (17), could be ameliorated by restoration of Lama2 in neonatal mice. P2 *dy^{2J}*/*dy^{2J}* mice were injected with AAV9-Cas9-sgRNA1 and -sgRNA2 via intraperitoneal route and compared to AAV9-GFP-treated controls (Fig. 3A).

RT-PCR analysis of gastrocnemius muscles isolated 10 weeks post-injection showed successful exon 2 inclusion (Fig. 3B). Quantitative RT-PCR indicated inclusion of exon 2 in 20% of *Lama2* transcripts (Fig. 3C). Consistent with RT-PCR analysis, western blot of muscle lysates demonstrated approximately 20% recovery of full-length Lama2 protein (Figs. 3D, 3E), which was located at the sarcolemmal membrane of the muscles, as indicated by immunofluorescence staining (Fig. 3F, left).

Histological examination using hematoxylin and eosin (H&E) and picrosirius red staining revealed an overall improved morphology of muscles with restored Lama2 expression (Fig. 3F, middle and right).

Morphometric analysis showed -50% reduction of fibrosis (Fig. 3G) and a 12 and 9% increase in cross-sectional area and minimum Feret's diameter, respectively, demonstrating an overall increase in muscle mass (Fig. 3L, 3M). A shift of distribution towards larger muscle fibers (Fig. 3N) and a decrease in number of centrally-located nuclei after treatment was also observed, although the trend did not reach statistical significance (Fig. 3H).

RT-PCR and immunofluorescence staining analyses of sciatic nerves showed no restoration of Lama2 transcript and protein (Figs. 3I, 3J). In line with this finding, assessment of general locomotion in these mice showed no significant improvement compared to the control *dy^{2J}*/*dy^{2J}* mice (Fig. 3K), as well as undetectable change of the extent of paralysis.

This finding recapitulated an earlier study demonstrating that muscle-specific overexpression of Laminin-211 in merosin-deficient mice improved muscle pathology, but not the associated paralysis (7), indicating that correction of the peripheral neuropathy requires restoration of Lama2 beyond skeletal muscles. The previously reported feasibility of CMV-driven gene expression mediated by AAV9 into sciatic nerves has been demonstrated mostly via vascular administration routes (26, 27).

### Example 5: Full-length Expression of Lama2 Post- temporal Vein Injection of AAV9-Cas9-sgRNA1 and -sgRNA2

To achieve a more robust tissue distribution, AAV9-Cas9-sgRNA1 and -sgRNA2 were injected into the temporal vein of P2 neonatal *dy^{2J}*/*dy^{2J}* mice (Fig. 4A). The temporal vein injection represents an alternative to tail vein for the systemic administration of AAV9 via blood circulation in young mice and has previously been shown to result in robust tissue distribution (27, 28).

At 30 weeks post-injection, the muscles and sciatic nerves were harvested and analyzed for targeted genomic DNA deletion and expression of full-length Lama2 mRNA and protein. The expected -493 bp genomic deletion was detected by PCR (Fig. 4N) and quantification by droplet digital PCR (ddPCR) revealed -3% editing on DNA level (Fig. 4O). Deep sequencing of these amplicons indicates that the majority of DSBs occurred at the intended sites and were properly joined by NHEJ mechanism, leading to 56.29% of perfect ligation, as shown in Table 2, which was effective to yield the inclusion of exon 2.

RT-PCR and western blot analyses of the TA muscles indicated expression of full-length Lama2 at transcript and protein levels (Fig. 4B-4E) of approximately 20 and 25%, respectively. Histological analysis showed expression of normally localized Lama2 protein at the sarcolemma (Fig. 4F), leading to significant reduction of fibrosis (Fig. 4G) and centrally located nuclei (Fig. 4H), as well as increase of minimum Feret's diameter and cross-sectional area (Figs. 4I, 4P, 4Q). Similar restoration is achieved in gastrocnemius (Fig. 5) and diaphragm (Fig. 6). However, there was no change in the number of inflammatory cells expressing Cd11b after treatment, presumably because the presence of Cd11b-positive cells in muscle fibers were considerably low to begin with (Fig. 7).

Lama2 restoration was also achieved in sciatic nerve at the transcript (Fig. 8A) and protein (Figs. 4J, 8B) levels. In order to assess the impact of Lama2 restoration on myelination, the sciatic nerves were stained with antibody against myelin binding protein (MBP). In the control-treated *dy^{2J}*/*dy^{2J},* presence of neurofilament-H-expressing axons lacking MBP expression was observed (Fig. 4J). In the treated mice, the axons were encapsulated by MBP, similar to the wild-type sciatic nerve, indicating improvement of the myelination defect.

Consequently, the degree of contracture and paralysis in the hind limbs and overall mobility of the AAV9-Cas9-sgRNA1 and -sgRNA2-treated mice were markedly improved compared to those of control *dy^{2J}*/*dy^{2J}* mice. Supporting this finding, open field test demonstrated significantly higher total distance travelled in the AAV9-Cas9-sgRNA1 and - sgRNA2-treated mice compared to the control AAV9-GFP-treated *dy^{2J}*/*dy^{2J}* (Fig. 4K). Moreover, the vertical activity, a trait that is almost never observed in the *dy^{2J}*/*dy^{2J}* mice (34), was detected in treated *dy^{2J}*/*dy^{2J}* mice at a near-wild-type level (Fig. 4L). Furthermore, the specific tetanic force measured using *in vivo* muscle analyzer was also restored towards the wild-type levels (Fig. 4M). Since the latter assay requires proper innervation of the muscles (29, 30), this data demonstrates that early restoration of Lama2 expression in both, peripheral nerves and skeletal muscles result in near-complete amelioration of muscle and nerve phenotypes.

In addition to the on-target activity, Cas9 nuclease has been shown to rarely generate DSB at non-intended off-target sites throughout the genome (16). Off-target effects are a safety concern for the eventual translation of gene editing therapies to humans. No off-target mutations at twenty potential off-target sites (ten for each sgRNA) in the mouse genome were observed (Fig. 9).

### Example 6: Bioinformatic Analysis of Targetable Exon/Intron Junctions and Intronic "GT" Motifs with Currently Available Cas9 Species.

Positions of all exon-intron junctions were obtained from GENCODE gene annotation gtf file (version 19, https://www.gencodegenes.org/releases/19.html) using customized scripts. 5' splice site (+1) mutations were extracted from the Human Gene Mutation Data database (HGMD, version 20140404). All of these mutations are pathogenic or disease-causing mutations (DM). As used herein, the first nucleotide of the intron is defined as the "+1" position. For each transcript, introns were extracted. For introns that were longer than 1000 base pairs, only the first 1000 base pairs were retained for analysis. For each gene, overlapping introns from different transcripts were merged. If a shorter intron overlaps with a larger one, then the shorter intron was used. For the introns containing a 5' splice site (+1) mutation, it was first examined if a "mutation-eliminating cut" site could be found. As used herein, a "mutation-eliminating cut" is defined as when any of the available Cas9s cleavage site occurring after the following PAM sequences: *NGG, NAG, NGA, NGCG, NNGRRT, NNNRRT, NNAGAA, NNNNRYAC, YG* could lead to a cutting that results in the elimination of the mutation. For mutations occurring at the "+1" position, the cut has to be made immediately upstream of the "+1" position ("cut at "+1"). The search for PAM sequences was done on both strands. For the introns where a "mutation-eliminating cut" could be found, the search continued into the introns and it was examined if a" "splice site-generating cut" site could be found. The first two nucleotides ("gt" at +1 and +2 sites) were required to be restored. Finally, "splice site-generating cut" sites that were at least 50 base pairs away from the next exon were filtered for, to avoid interference with the splicing machinery, although the splice site-generating cut site may be less than 50 base pairs away from the next exon (e.g., at least 25 base pairs away from a downstream (5' to 3') or adjacent exon).

### Splice Site Prediction

Splice sites were scored using (a) the MaxEntScan donor model, receiving in input 3 exonic bp and 6 intronic bp (22) centered at the splice site; (b) the DeepScan donor model, a proprietary predictor developed at Deep Genomics Inc., receiving in input 40 exonic bp and 40 intronic bp centered at the splice site. Both predictors were trained based on human annotated splice sites and genome sequence, and applied to the mouse sequence without further adjustments. MaxEntScan was previously shown to be one of the best performing publicly-available methods also for evaluating the deleteriousness of splice site altering variants (23). DeepScan is based on a deep neural network architecture (24) with convolutional layers and hidden layers consisting of rectified linear units trained using stochastic gradient descent to distinguish sequences of annotated splice sites from decoy splice sites found in introns; it achieved 98% area under the ROC curve for distinguishing held-out donor splice sites from held-out intronic sequences with the core donor dinucleotide GT, achieving a better performance than MaxEntScan as seen in Table 5:

**Table 5: Splice Site Prediction Scores Using DeepScan and MaxEntScan Donor Models**

| | Prediction Score threshold | N# GT sites ann* and pred** | % GT sites ann and pred | N# GT sites not-ann and pred | % GT pred and ann | % exons pred >= 1 GT site in <= 1kb | Avg pred GT site <=1kb per exon | Threshold explanation |
|---|---|---|---|---|---|---|---|---|
| Deep Scan DONOR | 0.0022 | 188,884 | 0.978 | 13,110,240 | 0.014 | 0.995 | 35.34 | Ultra sensitivity (recovers all experimentally-positive Lama2/Cas9 splice sites) |
| | 0.4800 | 173,761 | 0.900 | 1,165,522 | 0.130 | 0.864 | 12.85 | Optimal sensitivity (90% intronic 'gt' annotated as splice sites recovered) |
| | 0.9790 | 85,873 | 0.445 | 85,732 | 0.500 | 0.536 | 2.75 | Optimal specificity ("only" 50% intronic 'gt' not-annotated as splice sites recovered) |
| MaxEntScan DONOR | -20.0095 | 192,810 | 0.998 | 47,356,342 | 0.004 | 1.000 | 41.62 | Ultra sensitivity (same criteria) |
| | 4.9000 | 173,409 | 0.900 | 3,023,988 | 0.054 | 0.893 | 11.36 | Optimal sensitivity (same criteria) |

| | | | | | | | | |
|---|---|---|---|---|---|---|---|---|
| *) ann: annotated **) pred: predicted | | | | | | | | |

To derive genome wide statistics on donor splice site prediction, all intronic GT sites were extracted for GENCODE Basic version 19 transcripts, using the reverse complement of the hg19/b37 human reference genome sequence for negative strand transcripts; only sites for which the intronic categorization was not ambiguous were retained (i.e. intronic in all transcripts); sites were then categorized as annotated splice site donors or not. Finally, prediction models (MaxEntScan donor model, DeepScan donor model) were used to score these sites, and derive genome wide statistics at different prediction score thresholds. Exon statistics for reconstituted splice site donors (percentage of exons with at least one GT site within 1 kb predicted as donor when deleting the intervening intronic sequence and retaining the exon sequence, average number of such sites per exon) were calculated with respect to the longest transcript isoform of each gene in GENCODE Basic version 19.

### Example 7: Correction of a Second Splice Site Mutation in a Patient with MDC1A

A patient has two mutations in the *LAMA2* gene, with one of them being +1 mutation (c3037+1G>T) in 5'SS of exon 21 (Fig. 11A). By predicting potential splice sites in a nucleotide sequence an intron segment (having a mutation in a splice donor site) to be excised according to the methods described herein is identified. Excision of the identified intron segment (and the mutation) and simultaneous joining together of the DNA ends flanking the excised intron segment result in constitution of a functional donor splice site, such that the gene can now be properly spliced and a functional protein can be generated upon translation.

Using Human Splicing Finder (HSF) (http://www.umd.be/HSF3/) a "strength" of a non-mutated 5'ss was determined. For that, an exon 21 (181 nt) +50 intronic nucleotides (231 nt total) were analyzed by HSF (Fig. 11B). Sequence position 179 presents a strength for endogenous non-mutated 5'ss (=86.17) and MaxEnt score (=6.84) (Fig. 11C). For HSF score the threshold is established at 80, indicating that most human exons analyzed by this algorithm have a score 80 and above. The higher the score, the "stronger" the 5'ss. Similar, for MaxEnt score, the threshold is 3.0. The higher score, the stronger 5'ss prediction.

For mutated sequence (c3037+1G>T) HSF and MaxEnt scores dropped to 59.24 and -1.65, respectively, indicating that alteration of 5'ss will likely cause a splicing defect (Fig. 11C).

By modelling a double stranded break at any position within a sequence, a set of new exon/intron junctions are generated by removing an intervening sequence containing the mutation and linking the end of the exon 21 with intronic sequences starting with 'gt' in order to constitute a possible 5'ss. An example is shown in Figs. 11D and 11E. In Fig. 11D, the highlighted sequence is to be removed. In Fig. 11E, a model of a new exon/intron junction after the removal of sequence highlighted in Fig. 11D is shown. Table 6 shows the HSF scores and MaxEnt scores of various potential splices sites in the intronic sequence that could constitute a possible 5'ss.

**Table 6: HSF Scores and MaxEnt Scores of Potential Splices Sites in the Intronic Sequence that Could Constitute a Possible 5'ss for Exon 21 of the Lama2 Gene**

| | Name | Exonic sequence | Intronic sequences | HSF score | MaxEnt score |
|---|---|---|---|---|---|
| 1 | Control | GCTGGGACCTTTGGCCT ACAATCAGCAAGGGGCT GTGTTCCCTGCAACTGC AATTCTTTTGGGTCTAAG TCATTCGACTGTGAAGA GAGTGGACAATGTTGGT GCCAACCTGGAGTCACA GGGAAGAAATGTGACCG CTGTGCCCACGGCTATT TCAACTTCCAAGAAGGA GGCTGCACAG (SEQ ID NO: 44) | gtctgtaaatatgacttaag tcctacatattcactctgata gttttaggg (SEQ ID NO: 45) | 86.17 | 6.84 |
| 2 | Patient | | ttctgtaaatatgacttaagt cctacatattcactctgata gttttaggg (SEQ ID NO: 46) | **59.34** | **-1.65** |
| 3 | Del 4 nt | | gtaaatatgacttaagtcct acatattcactctgatagtttt agggtctg (SEQ ID NO: 47) | 99.44 | 8.76 |
| 4 | Del 19 nt | | gtcctacatattcactctgat agttttagggtctgacttctgt aacagtt (SEQ ID NO: 48) | 71.52 | 3.12 |
| 5 | De! 41 nt | | gttttagggtctgacttctgta acagtttgttcttttaaagga agcacag (SEQ ID NO: 49) | 71.52 | 0.68 |
| 6 | Del 49 nt | | gtctgacttctgtaacagttt gttcttttaaaggaagcaca gattgatac (SEQ ID NO: 50) | 83.84 | 5.06 |
| 7 | Del 60 nt | | gtaacagtttgttcttttaaa ggaagcacagattgatac aacgtatttaa (SEQ ID NO: 51) | 85.65 | 8.88 |
| 8 | Del 66 nt | | gtttgttcttttaaaggaagc acagattgatacaacgtatt taacataat (SEQ ID NO: 52) | 88.22 | 7.44 |
| 9 | Del 102 nt | | gtatttaacataatgagtaa tgtctacttatttttacgttactt aaaaaa (SEQ ID NO: 53) | 99.2 | 7.51 |
| 10 | Del 118 nt | | gtaatgtctacttatttttacgt tacttaaaaaatgaaatac atgtcagt (SEQ ID NO: 54) | 99.8 | 9.43 |
| 11 | Del 123 nt | | gtctacttatttttacgttactt aaaaaatgaaatacatgt cagtttgaa (SEQ ID NO: 55) | 71.83 | 0.92 |
| 12 | Del 139 nt | | gttacttaaaaaatgaaat acatgtcagtttgaaacact gccttaataac (SEQ ID NO: 56) | 85.93 | 0.49 |
| 13 | Del 162 nt | | gtcagtttgaaacactgcct taataactgtgtgcaagtac tcagtaaaaa (SEQ ID NO: 57) | 94.98 | 8.94 |
| 14 | Del 190 nt | | gtgtgcaagtactcagtaa aaatcagcagatgctcgttt ggttgtgtccc (SEQ ID NO: 58) | 82.38 | 6.46 |
| 15 | Del 192 nt | | gtgcaagtactcagtaaaa atcagcagatgctcgtttgg ttgtgtccc (SEQ ID NO: 59) | 85.42 | 2.83 |
| 16 | Del 198 nt | | gtactcagtaaaaatcagc agatgctcgtttggttgtgtc cc (SEQ ID NO: 60) | 96.36 | 7.04 |
| 17 | Del 205 nt | | gtaaaaatcagcagatgct cgtttggttgtgtccc (SEQ ID NO: 61) | 99.18 | 8.59 |
| 18 | Del 225 nt | | gtttggttgtgtccctccctgt ctgtgactttgattcagcca gtcactca (SEQ ID NO: 62) | 82.66 | 5.63 |
| 19 | Del 230 nt | | gttgtgtccctccctgtctgt gactttgattcagccagtca ctca (SEQ ID NO: 63) | 89.36 | -0.29 |
| 20 | Del 233 nt | | gtgtccctccctgtctgtgac tttgattcagccagtcactc a (SEQ ID NO: 64) | 83.19 | 3.16 |
| 21 | Del 235 nt | | gtccctccctgtctgtgacttt gattcagccagtcactca (SEQ ID NO: 65) | 74.16 | 0.68 |
| 22 | Del 245 nt | | gtctgtgactttgattcagcc agtcactcaaacactgcta cggcttgaaa (SEQ ID NO: 66) | 86.17 | 6.84 |
| 23 | Del 249 nt | | gtgactttgattcagccagt cactcaaacactgctacg gcttgaaa (SEQ ID NO: 67) | 89.22 | 6.6 |
| 24 | Del 267 nt | | gtcactcaaacactgctac ggcttgaaagttaaatgttc gatttttctgg (SEQ ID NO: 68) | 82.96 | 3.41 |
| 25 | Del 295 nt | | gttaaatgttcgatttttctgg taccagtcttatcttagtttaa gctcaa (SEQ ID NO: 69) | 81.28 | 2.54 |
| 26 | Del 302 nt | | gttcgatttttctggtaccagt cttatcttagtttaagctcaa (SEQ ID NO: 70) | 85.59 | 5.36 |
| 27 | Del 316 nt | | gtaccagtcttatcttagttta agctcaaaccccacgcca atgcatttca (SEQ ID NO: 71) | 99.62 | 7.88 |
| 28 | Del 322 nt | | gtcttatcttagtttaagctca aaccccacgccaatgcatt tca (SEQ ID NO: 72) | 71.52 | 0.67 |
| 29 | Del 333 nt | | gtttaagctcaaaccccac gccaatgcatttcaactttc cctttttccta (SEQ ID NO: 73) | 80.76 | 1.88 |
| 30 | Del 394 nt | | gtatccggatcaagaaaa gtcaaggtgtttttttttcctg gtacccccaa (SEQ ID NO: 74) | 100 | 7.91 |
| 31 | Del 412 | | gtcaaggtgtttttttttcctg gtaccccaaaaacatctttt catctac (SEQ ID NO: 75) | 80.88 | 2.98 |
| 32 | Del 418 nt | | gtgtttttttttcctggtaccc caaaaacatcttttcatcta c (SEQ ID NO: 76) | 83.62 | 2.28 |
| 33 | Del 420 nt | | gttttttttttcctggtacccca aaaacatcttttcatctac (SEQ ID NO: 77) | 84.35 | 2.93 |
| 34 | Del 435 nt | | gtaccccaaaaacatctttt catctacttttggctgccttg aaaactatg (SEQ ID NO: 78) | 99.39 | 8.63 |
| 35 | Del 487 nt | | gttctaagagagaaatag gttggctctagatatttagttc atcaaaaaat (SEQ ID NO: 79) | 83.18 | 0.74 |
| 36 | Del 505 nt | | gttggctctagatatttagttc atcaaaaaatgcaggcag aggtttctaa (SEQ ID NO: 80) | 5.52 | 88.07 |

A set of the generated junctions within the first 505 nucleotides in the intron are scored as described above (Table 6). For the new junction depicted in Fig. 11E, at position 179 HSF generates a score of 83.84 and MaxEnt score of 5.06, both above the given threshold for potentially functional 5'ss. These scores indicate that generation of this novel exon21/intron junction has a high probability of restoring functional splicing of the given exon. For the new junction depicted in Fig. 11E, at position 179 HSF generates a score of 83.84 and MaxEnt score of 5.06 (see number 6 del 49 nt) in Table 6.

Although the disclosure has been described with reference to certain specific aspects, various modifications thereof will be apparent to those skilled in the art. Any examples provided herein are included solely for the purpose of illustrating the disclosure and are not intended to limit the disclosure in any way. Any drawings provided herein are solely for the purpose of illustrating various aspects of the disclosure and are not intended to be drawn to scale or to limit the disclosure in any way.

### Documents Cited

1. Scotti, M. M., Swanson, M. S. RNA mis-splicing in disease. Nat Rev Genet 17, 19 (2016)
2. Bladen, C. L., Salgado, D., Monges, S. et al. The TREAT-NMD DMD Global Database: Analysis of More than 7,000 Duchenne Muscular Dystrophy Mutations. Hum Mutat 36, 395 (2015).
3. Ran, F. A., Cong, L., Yan, W. X. et al. In vivo genome editing using Staphylococcus aureus Cas9. Nature 520, 186 (2015).
4. Durbeej, M. Laminin-alpha2 Chain-Deficient Congenital Muscular Dystrophy: Pathophysiology and Development of Treatment. Curr Top Membr 76, 31 (2015).
5. Geranmayeh, F., Clement, E., Feng, L. H. et al. Genotype-phenotype correlation in a large population of muscular dystrophy patients with LAMA2 mutations. Neuromuscul Disord 20, 241 (2010).
6. Stenson, P. D., Mort, M., Ball, E. V. et al. The Human Gene Mutation Database: building a comprehensive mutation repository for clinical and molecular genetics, diagnostic testing and personalized genomic medicine. Hum Genet 133, 1 (2014).
7. Kuang, W., Xu, H., Vachon, P. H. et al. Merosin-deficient congenital muscular dystrophy. Partial genetic correction in two mouse models. J Clin Invest 102, 844 (1998).
8. Yang, Y., Wang, L., Bell, P. et al. A dual AAV system enables the Cas9-mediated correction of a metabolic liver disease in newborn mice. Nat Biotechnol 34, 334 (2016).
9. Yin, H., Xue, W., Chen, S. et al. Genome editing with Cas9 in adult mice corrects a disease mutation and phenotype. Nat Biotechnol 32, 551 (2014),
10. Yin, H., Song, C. Q., Dorkin, J. R. et al. Therapeutic genome editing by combined viral and non-viral delivery of CRISPR system components in vivo. Nat Biotechnol 34, 328 (2016).
11. Ferdousi, L., Rocheteau, P., Chayot, R. et al. More efficient repair of DNA double-strand breaks in skeletal muscle stem cells compared to their committed progeny. Stem Cell Research 13, 492 (2014).
12. Guiraud, S., Aartsma-Rus, A., Vieira, N. M. et al. The Pathogenesis and Therapy of Muscular Dystrophies. Annu Rev Genomics Hum Genet (2015)
13. Jinek, M., Chylinski, K., Fonfara, I. et al. A programmable dual-RNA-guided DNA endonuclease in adaptive bacterial immunity. Science 337, 816 (2012).
14. Cong, L., Ran, F. A., Cox, D. et al. Multiplex genome engineering using CRISPR/Cas systems. Science 339, 819 (2013).
15. Kim, S., Kim, D., Cho, S. W. et al. Highly efficient RNA-guided genome editing in human cells via delivery of purified Cas9 ribonucleoproteins. Genome Res 24, 1012 (2014).
16. Hendel, A., Bak, R. O., Clark, J. T. et al. Chemically modified guide RNAs enhance CRISPR-Cas genome editing in human primary cells. Nat Biotechnol (2015).
17. Mehuron, T., Kumar, A., Duarte, L. et al. Dysregulation of matricellular proteins is an early signature of pathology in laminin-deficient muscular dystrophy. Skelet Muscle 4, 14 (2014).
18. Kemaladewi, D. U., de Gorter, D. J., Aartsma-Rus, A. et al. Cell-type specific regulation of myostatin signaling. FASEB J 26, 1462 (2012).
19. Vilquin, J. T., Vignier, N., Tremblay, J. P. et al. Identification of homozygous and heterozygous dy2J mice by PCR. Neuromuscul Disord 10, 59 (2000).
20. Hsu, P. D., Lander, E. S., Zhang, F. Development and applications of CR!SPR-Cas9 for genome engineering. Cell 157, 1262 (2014).
21. Wojtal, D., Kemaladewi, D. U., Malam, Z. et al. Spell Checking Nature: Versatility of CRISPR/Cas9 for Developing Treatments for Inherited Disorders. Am J Hum Genet 98, 90 (2016).
22. Yeo, G., Burge, C. B. Maximum entropy modeling of short sequence motifs with applications to RNA splicing signals. J Comput Biol 11, 377 (2004).
23. Jian, X., Boerwinkle, E., Liu, X. In silico prediction of splice-altering single nucleotide variants in the human genome. Nucleic Acids Res 42, 13534 (2014).
24. LeCun, Y., Bengio, Y., Hinton, G. Deep learning. Nature 521, 436 (2015).
25. Roca, X., Krainer, A. R., Eperon, I. C. Pick one, but be quick: 5' splice sites and the problems of too many choices. Genes Dev 27, 129 (2013).
26. Tanguy, Y., Biferi, M. G., Besse, A. et al. Systemic AAVrh10 provides higher transgene expression than AAV9 in the brain and the spinal cord of neonatal mice. Front Mol Neurosci 8, 36 (2015).
27. Zincarelli, C., Soltys, S., Rengo, G. et al. Analysis of AAV serotypes 1-9 mediated gene expression and tropism in mice after systemic injection. Mol Ther 16, 1073 (2008).
28. Gombash Lampe, S. E., Kaspar, B. K., Foust, K. D. Intravenous injections in neonatal mice. J Vis Exp e52037 (2014).
29. Iyer, S. R., Valencia, A. P., Hernandez-Ochoa, E. O. et al. In Vivo Assessment of Muscle Contractility in Animal Studies. Methods Mol Biol 1460, 293 (2016).
30. Call, J. A., Lowe, D. A. Eccentric Contraction-Induced Muscle Injury: Reproducible, Quantitative, Physiological Models to Impair Skeletal Muscle's Capacity to Generate Force. Methods Mol Biol 1460, 3 (2016).
31. Tsai, S. Q., Zheng, Z., Nguyen, N. T. et al. GUIDE-seq enables genome-wide profiling of off-target cleavage by CRISPR-Cas nucleases. Nat Biotechnol 33, 187 (2015).
32. Sunada, Y., Bernier, S. M., Utani, A. et al. Identification of a novel mutant transcript of laminin alpha 2 chain gene responsible for muscular dystrophy and dysmyelination in dy2J mice. Hum Mol Genet 4, 1055 (1995).
33. Xu, H., Wu, X. R., Wewer, U. M. et al. Murine muscular dystrophy caused by a mutation in the laminin alpha 2 (Lama2) gene. Nat Genet 8, 297 (1994).
34. Tatem, K. S., Quinn, J. L., Phadke, A. et al. Behavioral and locomotor measurements using an open field activity monitoring system for skeletal muscle diseases. J Vis Exp 51785 (2014).
35. Hirano, H., Gootenberg, J. S., Horii, T. et al. Structure and Engineering of Francisella novicida Cas9. Cell 164, 950 (2016).
36. Kim, E., Koo, T., Park, S. W. et al. In vivo genome editing with a small Cas9 orthologue derived from Campylobacter jejuni. Nat Commun 8, 14500 (2017).
37. Kleinstiver, B. P., Prew, M. S., Tsai, S. Q. et al. Broadening the targeting range of Staphylococcus aureus CRISPR-Cas9 by modifying PAM recognition. Nat Biotechnol 33, 1293 (2015).
38. Kleinstiver, B. P., Prew, M. S., Tsai, S. Q. et al. Engineered CRISPR-Cas9 nucleases with altered PAM specificities. Nature 523, 481 (2015).

## Claims

1. A genome editing system, comprising at least one nuclease that generates blunt-ended DNA breaks in a sequence-specific manner,
wherein the genome editing system is configured to form a first and a second blunt-ended double strand break in an intron of a gene, wherein the intron comprises a splice donor site mutation that alters splice site recognition,
thereby excising a segment of the intron at a mutation-eliminating cut site of the intron and at a splice site-generating cut site of the intron of the gene and simultaneously joining DNA ends flanking the excised segment of the intron through non-homologous end joining to constitute a functional donor splice site;
wherein the at least one nuclease is:
a) a nuclease that recognizes a Protospacer Adjacent Motif (PAM) and wherein the system further comprises a mutation-eliminating single guide RNA (sgRNA) and a splice site-generating sgRNA or
b) a fusion polypeptide comprising a catalytically inactive nuclease that recognizes a PAM and a nuclease that generates blunt-ended DNA breaks.

2. A composition comprising at least one nuclease that generates blunt-ended DNA breaks in a sequence-specific manner,
wherein the composition is configured to form a first and a second blunt-ended double strand break in an intron of a gene, wherein the intron comprises a splice donor site mutation that alters splice site recognition,
thereby excising a segment of the intron at a mutation-eliminating cut site of the intron and at a splice site-generating cut site of the intron of the gene and simultaneously joining DNA ends flanking the excised segment of the intron through non-homologous end joining to constitute a functional donor splice site;
wherein the at least one nuclease is:
a) a nuclease that recognizes a Protospacer Adjacent Motif (PAM) and wherein the system further comprises a mutation-eliminating single guide RNA (sgRNA) and a splice site-generating sgRNA or
b) a fusion polypeptide comprising a catalytically inactive nuclease that recognizes a PAM and a nuclease that generates blunt-ended DNA breaks.

3. The system of claim 1, for use in constituting a functional donor splice site in a gene comprising a mutation in the donor splice site that alters splice site.

4. The system of claim 1 or 3 or composition of claim 2, wherein the excising of the segment of the intron and simultaneously joining the DNA ends flanking the excised segment of the intron to constitute a functional donor splice site results in restored splice site recognition in the intron.

5. The system or composition of any one of the preceding claims, wherein the nuclease is Cas9.

6. The system or composition of any one of the preceding claims, wherein the mutation occurs at the +1 position of the splice donor site.

7. The system or composition of any one of the preceding claims, wherein the system further comprises a delivery vehicle for delivery of the at least one nuclease that generates blunt-ended DNA breaks in a sequence-specific manner, and preferably wherein the delivery vehicle is an adeno-associated virus (AAV) vector, preferably an AAV9 vector.

8. The system or composition of any one of the preceding claims, wherein polynucleotides coding for the at least one nuclease that generates blunt-ended DNA breaks in a sequence-specific manner is in a vector, and preferably wherein the nuclease is Cas9 and the vector further comprises a mutation-eliminating single sgRNA or a splice site-generating sgRNA.

9. The system or composition of any one of the preceding claims, wherein the mutation is a pathogenic mutation.

10. The system or composition of any one of the preceding claims, wherein the gene is a gene listed in Table 1.

11. The system or composition of any one of the preceding claims wherein the gene is a *Lama2* gene and preferably wherein the mutation is a c.417+1 g→a mutation.

12. The system or composition of claim 10, wherein the mutation is in a subject having merosin deficient congenital muscular dystrophy (MDC1A).

13. The system or composition of any one of claims 1-8 or 11, for use in the treatment of merosin deficient congenital muscular dystrophy (MDC1A) in a subject.

## Patentansprüche

1. Genom-Editierungssystem, das mindestens eine Nuklease umfasst, die in sequenzspezifischer Weise stumpfendige DNA-Brüche erzeugt,
wobei das Genom-Editierungssystem so konfiguriert ist, dass es einen ersten und einen zweiten stumpfendigen Doppelstrangbruch in einem Intron eines Gens bildet, wobei das Intron eine Spleißdonorstellenmutation umfasst, die die Erkennung der Spleißstelle verändert,
wodurch ein Segment des Introns an einer mutationseliminierenden Schnittstelle des Introns und an einer spleißstellenerzeugenden Schnittstelle des Introns des Gens ausgeschnitten wird und gleichzeitig DNA-Enden, die das ausgeschnittene Segment des Introns flankieren, durch nicht-homologe Endverbindung verbunden werden, so dass eine funktionelle Donorspleißstelle erhalten wird;
wobei die mindestens eine Nuklease ist:
a) eine Nuklease, die ein Protospacer-Nachbarmotiv (PAM) erkennt, und wobei das System zudem eine mutationseliminierende single-Guide-RNA (sgRNA) und eine spleißstellenerzeugende sgRNA umfasst, oder
b) ein Fusionspolypeptid, umfassend eine katalytisch inaktive Nuklease, die ein PAM erkennt, und eine Nuklease, die stumpfendige DNA-Brüche erzeugt.

2. Zusammensetzung, umfassend mindestens eine Nuklease, die sequenzspezifisch stumpfendige DNA-Brüche erzeugt,
wobei die Zusammensetzung so konfiguriert ist, dass sie einen ersten und einen zweiten stumpfendigen Doppelstrangbruch in einem Intron eines Gens bildet, wobei das Intron eine Spleißdonorstellenmutation umfasst, die die Erkennung der Spleißstelle verändert,
wodurch ein Segment des Introns an einer mutationseliminierenden Schnittstelle des Introns und an einer spleißstellenerzeugenden Schnittstelle des Introns des Gens ausgeschnitten wird und gleichzeitig DNA-Enden, die das ausgeschnittene Segment des Introns flankieren, durch nicht-homologe Endverbindung verbunden werden, so dass eine funktionelle Donorspleißstelle erhalten wird
wobei die mindestens eine Nuklease ist:
a) eine Nuklease, die ein Protospacer-Nachbarmotiv (PAM) erkennt, und wobei das System zudem eine mutationseliminierende single-Guide-RNA (sgRNA) und eine spleißstellenerzeugende sgRNA umfasst, oder
b) ein Fusionspolypeptid, umfassend eine katalytisch inaktive Nuklease, die ein PAM erkennt, und eine Nuklease, die stumpfendige DNA-Brüche erzeugt.

3. System nach Anspruch 1 zur Verwendung bei der Bildung einer funktionellen Donorspleißstelle in einem Gen, das eine Mutation in der Donorspleißstelle umfasst, die die Spleißstelle verändert.

4. System nach Anspruch 1 oder 3 oder Zusammensetzung nach Anspruch 2, wobei das Ausschneiden des Segments des Introns und das gleichzeitige Verbinden der DNA-Enden, die das ausgeschnittene Segment des Introns flankieren, so dass eine funktionelle Donorspleißstelle erhalten wird, zu einer wiederhergestellten Spleißstellenerkennung im Intron führt.

5. System oder Zusammensetzung nach einem der vorhergehenden Ansprüche, wobei die Nuklease Cas9 ist.

6. System oder Zusammensetzung nach einem der vorhergehenden Ansprüche, wobei die Mutation an der +1-Position der Spleißdonorstelle auftritt.

7. System oder Zusammensetzung nach einem der vorhergehenden Ansprüche, wobei das System zudem ein Transportvehikel für den Transport der mindestens einen Nuklease umfasst, die stumpfendige DNA-Brüche in einer sequenzspezifischen Weise erzeugt, und wobei das Transportvehikel vorzugsweise ein Adeno-assoziierter Virus (AAV)-Vektor, vorzugsweise ein AAV9-Vektor, ist.

8. System oder Zusammensetzung nach einem der vorhergehenden Ansprüche, wobei Polynukleotide, die für die mindestens eine Nuklease codieren, die in sequenzspezifischer Weise stumpfendige DNA-Brüche erzeugt, in einem Vektor vorliegen, und wobei die Nuklease vorzugsweise Cas9 ist und der Vektor außerdem eine mutationseliminierende einzelne sgRNA oder eine spleißstellenerzeugende sgRNA umfasst.

9. System oder Zusammensetzung nach einem der vorhergehenden Ansprüche, wobei die Mutation eine pathogene Mutation ist.

10. System oder Zusammensetzung nach einem der vorhergehenden Ansprüche, wobei das Gen ein in Tabelle 1 aufgeführtes Gen ist.

11. System oder Zusammensetzung nach einem der vorhergehenden Ansprüche, wobei das Gen ein *Lama2*-Gen ist und wobei die Mutation vorzugsweise eine c.417+1 g→a-Mutation ist.

12. System oder Zusammensetzung nach Anspruch 10, wobei die Mutation in einem Individuum mit Merosin-defizienter kongenitaler Muskeldystrophie (MDC1A) vorliegt.

13. System oder Zusammensetzung nach einem der Ansprüche 1 bis 8 oder 11 zur Verwendung bei der Behandlung von Merosin-defizienter kongenitaler Muskeldystrophie (MDC1A) bei einem Individuum.

## Revendications

1. Système d'édition génomique, comprenant au moins une nucléase qui génère des ruptures d'ADN à extrémité franche d'une manière spécifique à une séquence,
le système d'édition génomique étant configuré pour former une première et une deuxième rupture de double brin à extrémité franche dans un intron d'un gène, l'intron comprenant une mutation du site donneur d'épissage qui modifie la reconnaissance du site d'épissage,
excisant ainsi un segment de l'intron au niveau d'un site de coupure éliminant la mutation de l'intron et au niveau d'un site de coupure générateur de site d'épissage de l'intron du gène et joignant simultanément des extrémités d'ADN flanquant le segment excisé de l'intron par assemblage d'extrémités non homologues pour constituer un site d'épissage de donneur fonctionnel ;
l'au moins une nucléase étant :
a) une nucléase qui reconnaît un motif adjacent au proto-espaceur (PAM) et le système comprenant en outre un ARN guide unique éliminant la mutation (ARNsg) et un ARNsg générant un site d'épissage ou
b) un polypeptide de fusion comprenant une nucléase catalytiquement inactive qui reconnaît un PAM et une nucléase qui génère des ruptures d'ADN à extrémité franche.

2. Composition comprenant au moins une nucléase qui génère des ruptures d'ADN à extrémité franche d'une manière spécifique à une séquence,
la composition étant configurée pour former une première et une deuxième rupture de double brin à extrémité franche dans un intron d'un gène, l'intron comprenant une mutation du site donneur d'épissage qui modifie la reconnaissance du site d'épissage,
excisant ainsi un segment de l'intron au niveau d'un site de coupure éliminant la mutation de l'intron et au niveau d'un site de coupure générateur de site d'épissage de l'intron du gène et joignant simultanément des extrémités d'ADN flanquant le segment excisé de l'intron par assemblage d'extrémités non homologues pour constituer un site d'épissage de donneur fonctionnel ;
l'au moins une nucléase étant :
a) une nucléase qui reconnaît un motif adjacent au proto-espaceur (PAM) et le système comprenant en outre un ARN à guide unique éliminant la mutation (ARNsg) et un site d'épissage générant un ARNsg ou
b) un polypeptide de fusion comprenant une nucléase catalytiquement inactive qui reconnaît un PAM et une nucléase qui génère des ruptures d'ADN à extrémité franche.

3. Système selon la revendication 1, pour une utilisation dans la constitution d'un site d'épissage de donneur fonctionnel dans un gène comprenant une mutation dans le site d'épissage de donneur qui modifie le site d'épissage.

4. Système selon la revendication 1 ou 3 ou composition selon la revendication 2, l'excision du segment de l'intron et simultanément la jointure des extrémités d'ADN flanquant le segment excisé de l'intron pour constituer un site d'épissage de donneur fonctionnel résultant en la reconnaissance du site d'épissage restauré dans l'intron.

5. Système ou composition selon l'une quelconque des revendications précédentes, la nucléase étant Cas9.

6. Système ou composition selon l'une quelconque des revendications précédentes, la mutation ayant lieu au niveau de la position +1 du site donneur d'épissage.

7. Système ou composition selon l'une quelconque des revendications précédentes, le système comprenant en outre un véhicule d'apport pour l'apport de l'au moins une nucléase qui génère des ruptures d'ADN à extrémité franche d'une manière spécifique à une séquence, et préférablement, le véhicule d'apport étant un vecteur de virus adénoassocié (AAV), préférablement un vecteur AAV9.

8. Système ou composition selon l'une quelconque des revendications précédentes, les polynucléotides codant pour l'au moins une nucléase qui génère des ruptures d'ADN à extrémité franche d'une manière spécifique à une séquence étant dans un vecteur, et préférablement la nucléase étant Cas9 et le vecteur comprenant en outre un ARNsg éliminant la mutation ou un ARNsg générant un site d'épissage.

9. Système ou composition selon l'une quelconque des revendications précédentes, la mutation étant une mutation pathogène.

10. Système ou composition selon l'une quelconque des revendications précédentes, le gène étant un gène listé dans le tableau 1.

11. Système ou composition selon l'une quelconque des revendications précédentes, le gène étant un gène *Lama2* et préférablement, la mutation étant une mutation c.417+1 g→a.

12. Système ou composition selon la revendication 10, la mutation étant dans un sujet atteint de dystrophie musculaire congénitale déficiente en mérosine (MDC1A).

13. Système ou composition selon l'une quelconque des revendications 1 à 8 ou 11, pour une utilisation dans le traitement d'une dystrophie musculaire congénitale déficiente en mérosine (MDC1A) chez un sujet.
